# EUROPEAN PATENT SPECIFICATION

(11) **EP 1 373 231 B1**
(45) Date of publication and mention of the grant of the patent: **08.06.2005**
(21) Application number: 02707045.7
(22) Date of filing: 18.03.2002
(51) Int. Cl.: C07D 271/06, A61K 31/4245, C07D 413/12, A61P 17/02

(54) **3-HETEROCYCLYL-PROPANO-HYDROXAMIC ACID AS PCP INHIBITORS**
3-HETEROCYCLYL PROPANOHYDROXAMSÄURE-DERIVATEN ALS PCP INHIBITOREN
DERIVES DE L'ACIDE 3-HETEROCYCLYL-PROPANOHYDROXAMIQUE EN TANT QU'INHIBITEURS DE LA PCP.

(30) Priority: 30.03.2001 GB 0108097
(43) Date of publication of application: 02.01.2004
(73) Proprietor: Pfizer Limited, Sandwich, Kent CT13 9NJ (GB); PFIZER INC., New York, N.Y. 10017 (US)
(72) Inventor: FISH, Paul Vincent, Sandwich, Kent CT13 9NJ (GB); KENDALL, Jackie Diane, Auckland (NZ); WHITLOCK, Gavin Alistair, Sandwich, Kent CT13 9NJ (GB); PFIZER INC., New York, NY 10017 (US)
(74) Representative: Motion, Keith Robert
(86) International application number: PCT/IB2002/000817
(87) International publication number: WO 2002/079175

(56) References cited:
- WO-A-00/37436
- WO-A-00/51993
- WO-A-01/47901

## Description

This invention relates to a certain class of compounds, and the pharmaceutically acceptable salts, solvates and prodrugs thereof, which inhibit Procollagen C-proteinase ("PCP"). They are therefore useful in the treatment of mammals having conditions alleviable by inhibition of PCP. Especially of interest is an antiscarring treatment for wounds.

Fibrotic tissues, including dermal scars, are characterised by excessive accumulation of extracellular matrix, mainly collagen type I. It is thought that inhibition of collagen deposition will reduce formation of scar tissue. Collagen is secreted as the precursor, procollagen, which is transformed into the insoluble collagen by cleavage of the C-terminal propeptide by PCP. PCP is a zinc-dependent metalloprotease which is secreted from TGF-β-activated fibroblasts belonging to the subfamily of astacin-like proteases and able to cleave the C-terminal peptide of types I, II and III procollagens. Furthermore, data suggest that PCP activates lysyl oxidase, an enzyme essential for the formation of covalent cross-links which stabilise the fibrous form of collagen. Therefore, inhibition of PCP may not only reduce collagen deposition but may also make collagen more accessible for degradation.

Collagen is integral to, among other things, the proper formation of connective tissue. Thus, the over- or under-production of collagen or the production of abnormal collagen (including incorrectly processed collagen) has been linked with numerous connective tissue diseases and disorders. Mounting evidence suggests that PCP is an essential key enzyme for the proper maturation of collagen (see for example international Patent Application publication number WO 97/05865).

The present invention relates to substances capable of inhibiting PCP activity in order to regulate, modulate and/or reduce collagen formation and deposition. More specifically, the invention relates to the use of compounds and pharmaceutical compositions thereof for the treatment of various conditions relating to production of collagen.

At present more than nineteen types of collagens have been identified. These collagens, including fibrillar collagen types I, II, III are synthesized as procollagen precursor molecules which contain amino- and carboxy-terminal peptide extensions. These peptide extensions, referred to as "pro-regions," are designated as N- and C- propeptides, respectively.
The pro-regions are typically cleaved upon secretion of the procollagen triple helical precursor molecule from the cell to yield a mature triple helical collagen molecule. Upon cleavage, the "mature" collagen molecule is capable of association, for example, into highly structured collagen fibers. See e.g., Fessler and Fessler, 1978, Annu. Rev. Biochem. 47:129-162; Bomstein and Traub, 1979, in: The Proteins (eds. Neurath, H. and Hill, R.H.), Academic Press, New York, pp. 412-632; Kivirikko et al., 1984, in: Extracellur Matrix Biochemistry (eds. Piez, K.A. and Reddi. A.H.), Elsevier Science Publishing Co., Inc., New York, pp. 83-118; Prockop and Kivirikko, 1984, N. Engl, J. Med. 311:376-383; Kuhn, 1987, in: Structure and Function of Collagen Types (eds. Mayne, R. and Burgeson, R.E.), Academic Press, Inc., Orlando, Florida, pp. 1-42.

An array of conditions has been associated with the inappropriate or unregulated production of collagen, including pathological fibrosis or scarring, including endocardial sclerosis, idiopathic interstitial fibrosis, interstitial pulmonary fibrosis, perimuscular fibrosis, Symmers' fibrosis, pericentral fibrosis, hepatitis, dermatofibroma, cirrhosis such as billary cirrhosis and alcoholic cirrhosis, acute pulmonary fibrosis, idiopathic pulmonary fibrosis, acute respiratory distress syndrome, kidney fibrosis/glomerulonephritis, kidney fibrosis/diabetic nephropathy, scleroderma/systemic, scleroderma/local, keloids, hypertrophic scars, severe joint adhesions/arthritis, myelofibrosis, corneal scarring, cystic fibrosis, muscular dystrophy (duchenne's), cardiac fibrosis, muscular fibrosis/retinal separation, esophageal stricture and Pyronie's disease. Further fibrotic disorders may be induced or initiated by surgery, including scar revision/plastic surgeries, glaucoma, cataract fibrosis, corneal scarring, joint adhesions, graft vs. host disease, tendon surgery, nerve entrapment, dupuytren's contracture, OB/GYN adhesions/fibrosis, pelvic adhesions, peridural fibrosis, restenosis. Other conditions where collagen plays a key role include bums. Fibrosis of lung tissue is also observed in patients suffering from chronic obstructive airways disease (COAD) and asthma. One strategy for the treatment of these diseases and conditions is to inhibit the overproduction and/or deposition and/or unregulation of collagen. Thus, identification and isolation of molecules which control, inhibit and/or modulate the production and deposition of collagen are of major medical interest.

Recent evidence suggests that PCP is the essential key enzyme that catalyzes the cleavage of the Procollagen C-propeptide. This has been demonstrated in fibrillar collagens, including type I, type II, and type III collagen.

PCP was first observed in the culture media of human and mouse fibroblasts (Goldberg et al., 1975, Cell 4:45-50; Kessler and Goldberg, 1978, Anal. Biochem. 86:463-469), and chick tendon fibroblasts (Duskin et al., 1978, Arch. Biochem. Biophys. 185:326-332; Leung et al., 1979, J. Biol, Chem. 254:224-232). An acidic proteinase which removes the C-terminal propeptides from type I procollagen has also been identified (Davidson et al., 1979, Eur. J. Biochem. 100:551).

A partially purified protein having PCP activity was obtained from chick calvaria in 1982. Njieha et al., 1982, Biochemistry 23:757-764. In 1985, chicken PCP was isolated, purified and characterized from conditioned media of chick embryo tendons. Hojima et al., 1985, J. Biol. Chem. 260:15996-16003. Murine PCP has been subsequently purified from media of cultured mouse fibroblasts. Kessler et al., 1986, Collagen Relat. Res. 6:249-266; Kessler and Adar, 1989, Eur. J. Biochem. 186:115-121. Finally, the cDNA encoding human PCP has been identified, as set forth in the above-referenced articles and references disclosed therein.

Experiments conducted with these purified forms of chick and mouse PCP have indicated that the enzyme is instrumental In the formation of functional collagen fibers. Fertala et al., 1994, J. Biol. Chem. 269:11584.

As a consequence of the enzyme's apparent importance to collagen production, scientists have identified a number of PCP inhibitors. See e.g., Hojima et al., supra. For example, several metal chelators have demonstrated activity as PCP inhibitors. Likewise, chymostatin and pepstatin A were found to be relatively strong inhibitors of PCP. Additionally, α₂-Macroglobuline, ovostatin, and fetal bovine serum appear to at least partially inhibit PCP activity.

Dithiothreitol, SDS, concanavalin A, Zn²⁺, Cu²⁺, and Cd²⁺ are similarly reported to be inhibitory at low concentrations. Likewise, some reducing agents, several amino acids, phosphate, and ammonium sulfate were inhibitory at concentrations of 1-10mM. Further, the enzyme was shown to be inhibited by the basic amino acids lysine and arginine (Leung et al., supra; Ryhänen et al., 1982, Arch. Biochem. Biophys. 215:230-235). Finally, high concentrations of NaCl or Tris-HCl buffer were found to inhibit PCP's activity. For example, it is reported that, with 0.2, 0.3, and 0.5M NaCl, the activity of PCP was reduced 66, 38, and 25%, respectively, of that observed with the standard assay concentration of 0.15M. Tris-HCl buffer in a concentration of 0.2-0.5M markedly inhibited activity (Hojima et al., supra). PCP activity and its inhibition have been determined using a wide array of assays. See e.g., Kessler and Goldberg, 1978, Anal. Biochem. 86:463; Njieha et al., 1982, Biochemistry 21:757-764. As articulated in numerous publications, the enzyme is difficult to isolate by conventional biochemical means and the identity of the cDNA sequence encoding such enzyme was not known until reported in the above referenced and related patent applications.

In view of its essential role in the formation and maturation of collagen PCP appears to be an ideal target for the treatment of disorders associated with the inappropriate or unregulated production and maturation of collagen. However, none of the inhibitors so far disclosed has proven to be an effective therapeutic for the treatment of collagen-related diseases and conditions.

The identification of effective compounds which specifically inhibit the activity of PCP to regulate and modulate abnormal or inappropriate collagen production is therefore desirable and the object of this invention.

Matrix metalloproteases (MMPs) constitute a family of structurally similar zinc-containing metalloproteases, which are involved in the remodelling, repair and degradation of extracellular matrix proteins, both as part of normal physiological processes and in pathological conditions.

Another important function of certain MMPs is to activate other enzymes, including other MMPs, by cleaving the pro-domain from their protease domain. Thus, certain MMPs act to regulate the activities of other MMPs, so that over-production in one MMP may lead to excessive proteolysis of extracellular matrix by another, e.g. MMP-14 activates pro-MMP-2

During the healing of normal and chronic wounds, MMP-1 is expressed by migrating keratinocytes at the wound edges (U.K. Saarialho-Kere, S.O. Kovacs, A.P. Pentiand, J. Clin. Invest. 1993, 92, 2858-66). There is evidence which suggests MMP-1 is required for keratinocyte migration on a collagen type I matrix in vitro, and is completely inhibited by the presence of the non-selective MMP inhibitor SC44463 ((N4-hydroxy)-N1-[(1S)-2-(4-methoxyphenyl)methyl-1-((1R)-methylamino)carbonyl)]-(2R)-2-(2-methylpropyl)butanediamide) (B.K. Pilcher, J.A. Dumin, B.D. Sudbeck, S.M. Krane, H.G. Welgus, W.C. Parks, J. Cell Biol., 1997, 137, 1-13). Keratinocyte migration in vivo is essential for effective wound healing to occur.

MMP-2 and MMP-9 appear to play important roles in wound healing during the extended remodelling phase and the onset of re-epithelialisation, respectively (M.S. Agren, Brit. J. Dermatology, 1994, 131, 634-40; T. Salo, M. Mäkänen, M. Kylmäniemi, Lab. Invest., 1994, 70, 176-82). The potent, non-selective MMP inhibitor BB94 ((2S,3R)-5-methyl-3-{[(1S)-1-(methylcarbamoyl)-2-phenylethyl]carbamoyl}-2-[(2-thienylthio)methyl]hexanohydroxamic acid, batimastat), inhibits endothelial cell invasion of basement membrane, thereby inhibiting angiogenesis (G. Tarboletti, A. Garofalo, D. Belotti, T. Drudis, P. Borsotti, E. Scanziani, P.D. Brown, R. Giavazzi, J. Natl. Cancer Inst., 1995, 87, 293-8). There is evidence that this process requires active MMP-2 and/or 9.

Thus PCP inhibitors which significantly inhibit MMPs 1 and/or 2 and/or 9 would be expected to impair wound healing. MMP-14 is responsible for the activation of MMP-2, and thus inhibition of MMP-14 might also result in impaired wound healing.

For recent reviews of MMPs, see Zask et al, Current Pharmaceutical Design, 1996, 2, 624-661; Beckett, Exp. Opin. Ther. Patents, 1996, 6,1305-1315; and Beckett et al, Drug Discovery Today, vol 1 (no.1), 1996, 16-26.

Alternative names for various MMPs and substrates acted on by these are shown in the table below (Zask et al, supra).

| Enzyme | Other names | Preferred substrates |
|---|---|---|
| MMP-1 | Collagenase-1, interstitial collagenase | Collagens I, II, III, VII, X, gelatins |
| MMP-2 | Gelatinase A, 72kDa gelatinase | Gelatins, collagens IV, V, VII, X, elastin, fibronectin; activates pro-MMP-13 |
| MMP-3 | Stromelysin-1 | Proteoglycans, laminin, fibronectin, gelatins. |
| MMP-7 | Pump, Matrilysin | Proteoglycans, laminin, fibronectin, gelatins, collagen IV, elastin, activates pro-MMP-1 and -2. |
| MMP-8 | Collagenase-2, neutrophil collagenase | Collagens I, II, III |
| MMP-9 | Gelatinase B, 92 kDa gelatinase | Gelatins, collagens IV, V, elastin |
| MMP-12 | Macrophage metalloelastase | Elastin, collagen IV, fibronectin, activates pro-MMP-2 & 3. |
| MMP-13 | Collagenase-3 | Collagens I, II, III, gelatins |
| MMP-14 | MT-MMP-1 | Activates pro-MMP-2 & 13, gelatins |
| MMP-15 | MT-MMP-2 | unknown |
| MMP-16 | MT-MMP-3 | Activates pro-MMP-2 |
| MMP-17 | MT-MMP-4 | unknown |

International Patent Applications PCT/IB00/01855 (published as WO 01/47901) and PCT/IB01/02360 (filed on 7^{th} December 2001), and foreign equivalents thereof, describe various 3-heterocyclylpropanohydroxamic acid PCP inhibitors. The teachings of both of these are herein incorporated by reference in their entirety.

According to one aspect of the present invention, there are provided compounds of formula (I): 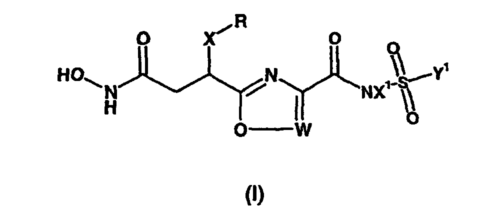 wherein:
X is C₁₋₆ alkylene, or C₂₋₆ alkenylene, each of which is optionally substituted by one or more fluorine atoms,
R is aryl, C₃₋₈ cycloalkyl or C₅₋₈ cycloalkenyl optionally substituted by one or more fluorine atoms,
W is N or CZ,
Z is H, or C₁-C₄ alkyl optionally substituted with halogen,
X¹ is independently H or C₁-C₄ alkyl,
Y¹ is independently
   C₁-C₄ alkyl, optionally substituted by aryl, or by one or more halogen atoms, with the proviso that when Y¹ is methyl, X¹ is not H,
or Y¹ is independently aryl, or a mono or bicyclic non-aromatic carbocyclic or heterocyclic moiety containing up to 10 ring atoms and which can include up to 3 ring heteroatoms, independently selected from N, O and S, which ring moiety is optionally substituted by one or more substituents independently selected from halogen, C₁-C₄ alkoxy or C₁-C₄ alkyl optionally substituted by one or more halogen,
and the pharmaceutically acceptable salts and solvates (including hydrates) thereof.

"Alkyl", "alkylene", "alkoxy", and "alkenylene" groups, including groups incorporating said moieties, may be straight chain or branched where the number of carbon atoms allows.

"Aryl" is a mono or bicyclic aromatic carbocyclic or heterocyclic moiety containing up to 10 ring atoms, and which can include up to 3 ring heteroatoms, selected from N, O and S, which ring moiety is optionally substituted by one or more substituents, independently selected from halogen, C₁-C₄ alkoxy, C₁-C₄ alkyl optionally substituted by one or more halogen.

Halogen is taken to mean fluorine, chlorine, bromine or iodine.

Pharmaceutically-acceptable salts are well known to those skilled in the art, and for example include those mentioned in the art cited above, and by Berge et al, in J.Pharm.Sci., 66, 1-19 (1977). Suitable acid addition salts are formed from acids which form non-toxic salts and include the hydrochloride, hydrobromide, hydroiodide, nitrate, sulphate, bisulphate, phosphate, hydrogenphosphate, acetate, trifluoroacetate, gluconate, lactate, salicylate, citrate, tartrate, ascorbate, succinate, maleate, fumarate, gluconate, formate, benzoate, methanesulphonate, ethanesulphonate, benzenesulphonate, pamoate, camsylate, and p-toluenesulphonate salts.

Pharmaceutically acceptable base addition salts are well known to those skilled in the art, and for example include those mentioned in the art cited above, and can be formed from bases which form non-toxic salts and include the aluminium, calcium, lithium, magnesium, potassium, sodium and zinc salts, and salts of non-toxic amines such as diethanolamine.

Certain of the compounds of formula (I) may exist in one or more zwitterionic forms. It is to be understood that pharmaceutically acceptable salts includes all such zwitterions.

Certain of the compounds of formula (I), their salts, solvates, prodrugs, etc. may exist in one or more polymorphic forms. It is to be understood that the invention includes all such polymorphs.

The compounds of formula (I), their salts, hydrates, prodrugs etc. can exhibit isotopic variation, e.g. forms with enriched ²H, ³H, ¹³C, ¹⁴C, ¹⁵N, ¹⁸O, etc. may be prepared, for example by suitable variation of the synthetic methods described herein using methods and reagents known in the art or routine modification thereof. All such isotopic variants are included in the scope of the invention.

Prodrug moieties are well-known to those skilled in the art (see for example the article by H Feres, in Drugs of Today, vol 19, no.9 (1983) pp.499-538, especially section A1), and for example include those specifically mentioned in A.A. Sinkula's article in Annual Reports in Medicinal Chemistry, vol 10, chapter 31, pp.306-326, herein incorporated by reference, and the references therein. Specific prodrug moieties which may be specifically mentioned are aliphatic-aromatic, carbonate, phosphate and carboxylic esters, carbamates, peptides, glycoside, acetals and ketals, tetrahydropyranyl and silyl ethers. Such prodrug moieties can be cleaved in situ, e.g. are hydrolysable in physiological conditions, to give compounds of formula (I).

Certain of the compounds of the formula (I) may exist as geometric isomers. Certain of the compounds of the formula (I) may exist in one or more tautomeric forms. The compounds of the formula (I) may possess one or more asymmetric centres, apart from the specified centres in formula (I), and so exist in two or more stereoisomeric forms. The present invention includes all the individual stereoisomers, tautomers and geometric isomers of the compounds of formula (I) and mixtures thereof.

Preferably the compounds of formula (I) have the following stereochemistry (IA):

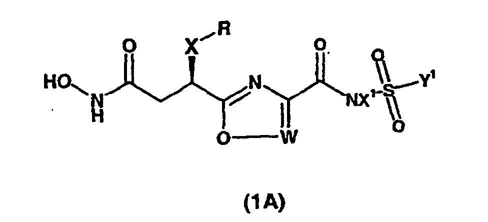

Preferably X is a linear C₂₋₄ alkylene moiety optionally substituted by one or more fluorine atoms.
Most preferably X is propylene.

Preferably R is C₃₋₈ cycloalkyl optionally substituted by one or more fluorine atoms.
More preferably R is cyclobutyl or cyclohexyl optionally substituted by one or more fluorine atoms.
Yet more preferably R is cyclobutyl or cyclohexyl.
Most preferably R is cyclohexyl.

Preferably W is N, CH or CCH₃.
Most preferably W is N.

Preferably Y¹ is C₁-C₄ alkyl optionally substituted by phenyl or by one or more halogen atoms, or Y¹ is phenyl, optionally substituted by one or more substituents independently selected from halogen, C₁-C₄ alkoxy, C₁-C₄ alkyl optionally substituted with one or more halogen, and which phenyl ring is optionally pyrido-fused, or Y¹ is a 5-or 6- membered heterocyclic ring, which can include one or two heteroatoms selected from N, O and S, which heterocyclic ring is optionally substituted by one or more substituents independently selected from halogen, C₁-C₄ alkoxy, C₁-C₄ alkyl optionally substituted with one or more halogen.

More preferably Y¹ is phenyl, 4-methylphenyl, 4-methoxyphenyl, 4-fluorophenyl, 4-isopropylphenyl, 3,4-dimethoxyphenyl, 8-quinolinyl, 3,5-dimethyl-4-isoxazolyl, isopropyl, methyl, benzyl or 3-pyridyl.

Even more preferably Y¹ is phenyl, benzyl, 3,4-dimethoxyphenyl, or pyridyl.

Most preferably Y¹ is phenyl.

Preferably X¹ is H or methyl.
Most preferably X¹ is H.

A preferred group of compounds is that in which each substituent is as specified in the Examples below.

Another preferred group of compounds are those of the examples below and the salts, and solvates thereof.

Another aspect of the invention is a compound of formula (I) described herein, and the salts and solvates, for use in medicine.

Another aspect of the invention is a compound of formula (I) described herein, and the salts and solvates thereof, for use as medicament for the treatment of a PCP-mediated condition or disease.

Another aspect of the invention is the use of a compound of formula (I) described herein, and the salts and solvates thereof, in the manufacture of an antiscarring medicament.

Another aspect of the invention is the use of a compound of formula (I) described herein, and the salts and solvates thereof, in the manufacture of a medicament for the treatment of a PCP-mediated condition or disease.

Another aspect of the invention is a pharmaceutical composition comprising a compound of formula (I), salts thereof, solvates thereof, and a pharmaceutically acceptable diluent, carrier or adjuvant.

It is to be appreciated that reference to treatment includes prophylaxis as well as the alleviation of established symptoms of PCP-mediated conditions and diseases.

The invention further provides Methods for the production of compounds of the invention, which are described below and in the Examples and Preparations. The skilled man will appreciate that the compounds of the invention could be made by methods other than those specifically described herein, by adaptation of the methods herein described in the sections below and/or adaptation thereof, for example by methods known in the art. Suitable guides to synthesis, functional group transformations, use of protecting groups, etc. are, for example, "Comprehensive Organic Transformations" by RC Larock, VCH Publishers Inc. (1989), "Advanced Organic Chemistry" by J March, Wiley Interscience (1985), "Designing Organic Synthesis" by S Warren, Wiley Interscience (1978), "Organic Synthesis - The Disconnection Approach" by S Warren, Wiley Interscience (1982), "Guidebook to Organic Synthesis" by RK Mackie and DM Smith, Longman (1982), "Protective Groups in Organic Synthesis" byTW Greene and PGM Wuts, John Wiley and Sons Inc. (1999), and PJ Kocienski, in "Protecting Groups", Georg Thieme Verlag (1994), and any updated versions of said standard works.

In the Methods below, unless otherwise specified, the substituents are as defined above with reference to the compounds of formula (I) above.

The compounds of formula (I), where W is N, can be prepared according to the scheme below:

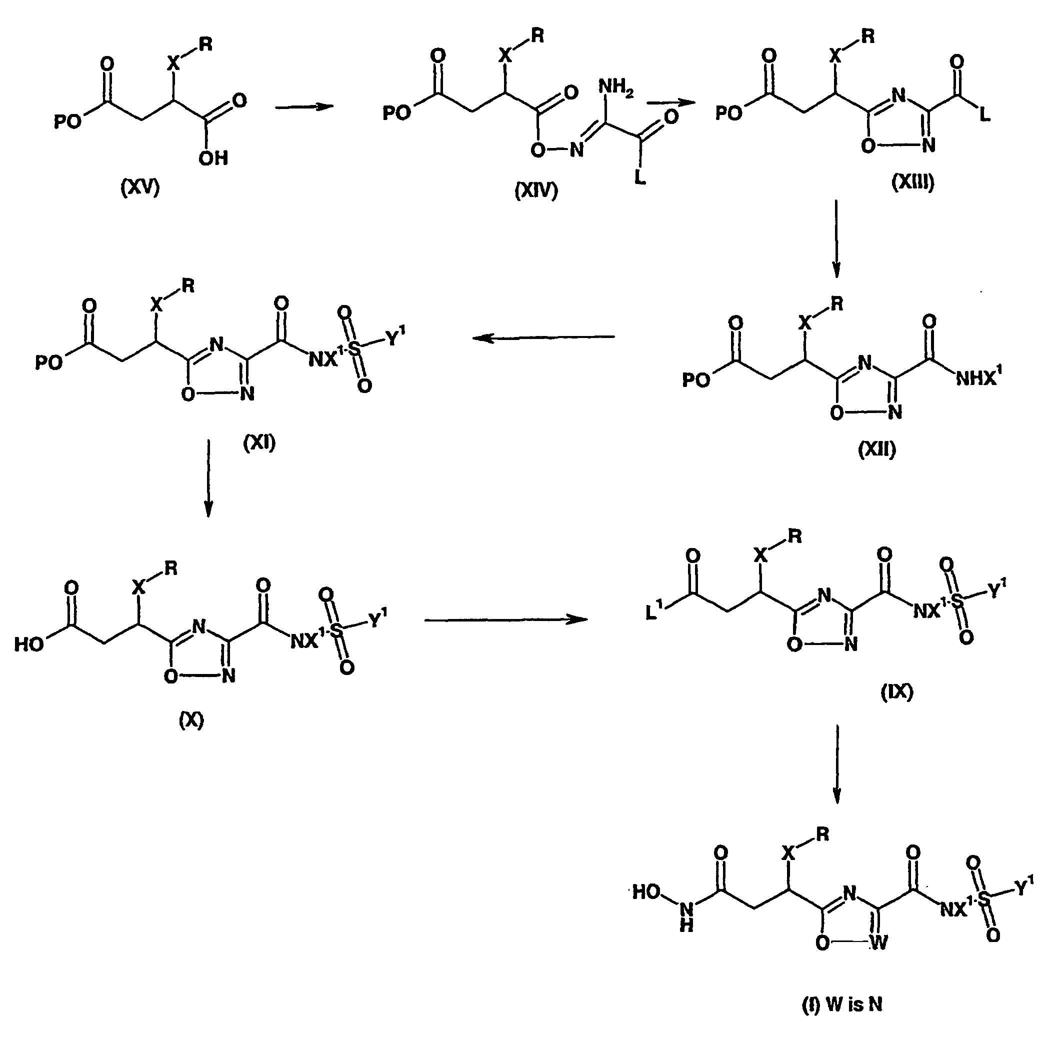

The compounds of formula (I), where W is CZ, can be prepared according to the scheme below:

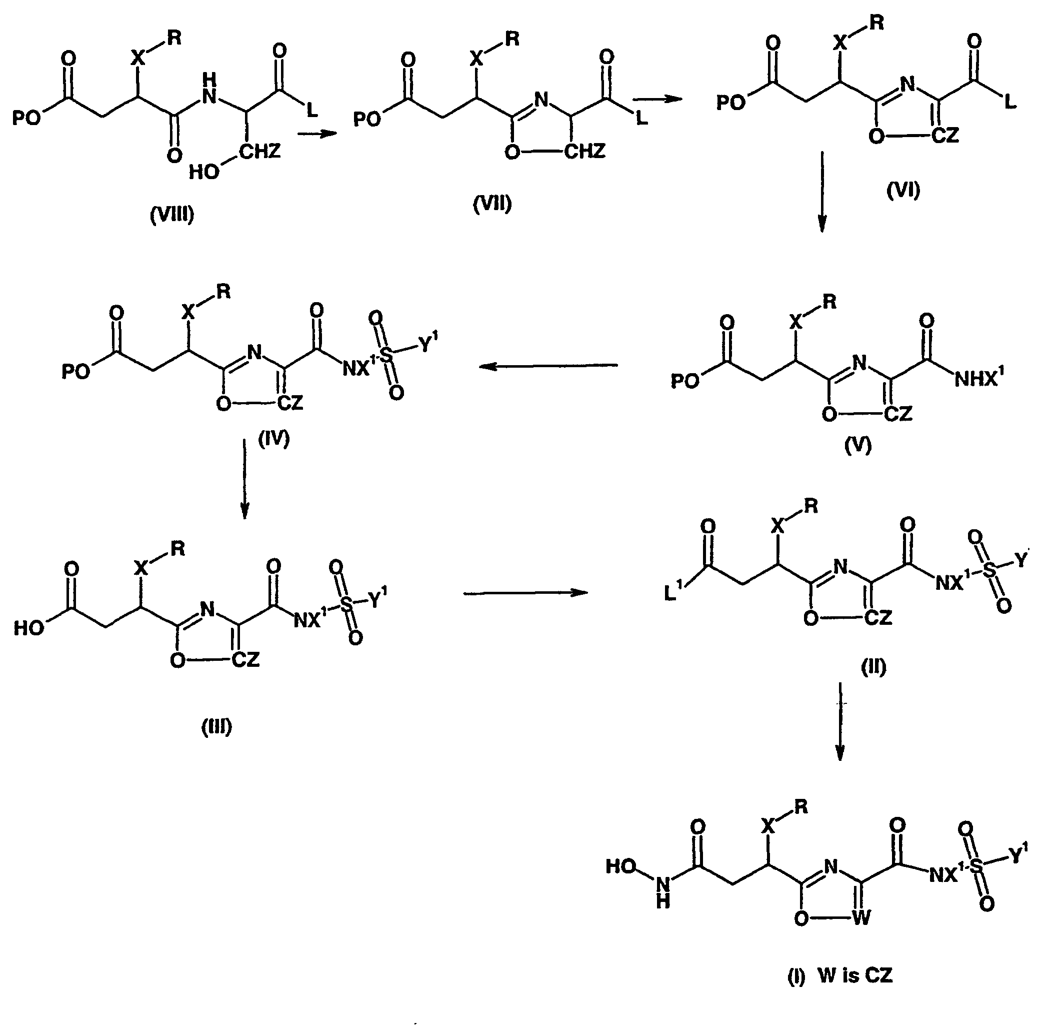

The hydroxamic acid compounds of formula (I) can be made by reaction of the corresponding activated acid derivatives of formula (II) or (IX), where L¹ is a suitable leaving group, with hydroxylamine optionally protected with a suitable O-protecting group, such as *O*-trimethyl silyl group, which can be removed after the substitution reaction with methanol, as illustrated in Example 1.

Suitable leaving groups are generally those which would leave in a more efficient manner than the hydroxide of the parent acids (III) or (X), in a nucleophilic substitution reaction, such as an anhydride, or imidazole. Other suitable leaving groups are familiar to those working in the field of amino acid coupling.

Such compounds of formula (II) or (IX) may be made via standard chemistry from the corresponding acids (X) or (III). Compounds of formula (II) or (IX) where L¹ is a leaving group such as anhydride or imidazole and the like, can be made from the corresponding compounds of formula (X) or (III) by conventional methods, including methods typified in e.g. Examples 1, 2 and 10. These examples illustrate reaction of the acid with an amine base followed by addition of an alkylhaloformate to form a compound of formula (IX) or (II) wherein L¹ is alkylOCO₂ leaving group L¹, in a suitable solvent such as tetrahydrofuran. Example 3 illustrates the use of a coupling agent such as carbonyldiimidazole to form the imidazolide intermediate, with an imidazole leaving group L¹.

Other methods of making hydroxamic acids (I) are known and may be used, e.g. those mentioned in the text by J.March, *supra,* chapters 0-54, 0-57 and 6-4, and relevant references therein.

Acids of formula (III) or (X) may be made by deprotection of the O-protected species of formula (IV) or (XI). Suitable O-protecting groups can be found in the chapter on O-protection in the book by Greene and Wuts, *supra,* and include C₁₋₄ alkoxy such as t-butoxy, benzyloxy, trialkylsilyloxy such as trimethylsilyloxy, etc..
The deprotection method is determined by the protective group used, as is well known in the art (see Greene and Wuts, *supra*). E.g. benzyl groups may be removed by hydrogenation, suitably using a catalytic transfer hydrogenation method, t-butyl groups may be removed by treatment with an acid such as trifluoroacetic acid (as typified in Preparation 2), etc.

Compounds of formula (IV) or (XI) can be made by reaction of compounds of formula (V) or (XII), deprotonated if necessary with a base, with a suitable reagent of formula X²SO₂Y¹, where X² is a suitable leaving group such as chloride or bromide in a nucleophillic substitution reaction, as typified in Preparations 1 and 3.

Compounds of formula (V) and (XII) may be made by addition of an amine of formula NH₂X¹ to a compound of formula (VI) or (XIII) to displace L, where L is a suitable leaving group such as methoxy or ethoxy, as exemplified in Preparation 28. Other examples of such standard nucleophillic acyl substitution reactions are well known to those skilled in the art; further examples can be found in references such as: Trost, B. M., Fleming, I., Heathcock, C. H. Comprehensive Organic Synthesis, New York, 1991, vol 6 and Larock, R. C. Comprehensive Organic Transformations, Wiley-VCH, New York, 1999.

Where W=N:

Compounds of formula (XIII), e.g. where P is a t-butoxy group, can be made for example by condensation reaction of a corresponding compound of formula (XIV), for example by heating to elevated temperature in an inert solvent such as in xylene at about 130°C, this reaction being typified by Preparation 27.

Compounds of formula (XIV) can be made for example by coupling an acid of formula (XV) with a reagent of formula C(NH₂)(COL)=NOH, which is available via literature methods or adaptation thereof in a conventional manner, such as typified in Preparation 26. Typically the condensation is carried out by adding a solution of the acid (XV) in a suitable inert solvent such as 1,4-dioxane to a suitable agent such as 1-hydroxybenzotriazole hydrate, followed by addition of a suitable coupling agent such as a carbodiimide coupling agent, e.g. N,N'-dicyclohexylcarbodiimide, then treatment with the reagent C(NH₂)(COL)=NOH. Suitably the coupling is carried out at ambient temperature.

Compounds of formula (XV) can be made by hydrogenation of the corresponding itaconate derivative, which in turn can be made by conventional methods such as the Stobbe condensation. The preparation of these Intermediates is exemplified in Preparation 25.

Where W=CZ:

Compounds of formula (VI), e.g. where P is a t-butoxy group, can be made for example by oxidation of a compound of formula (VII). Suitably the oxidation is carried out using copper (II) bromide with hexamethylenetetramine and a base such as DBU. The reagents, conditions, etc. are typified in Preparation 32 below.

Compounds of formula (VII) may be made by condensation of compounds of formula (VIII), for example by treatment of the compound of formula (VIII) with a suitable agent such as Burgess Reagent, in an anhydrous solvent such as THF, as exemplified in Preparation 31.

Compounds of formula (VIII) may be made by condensation of the acid of formula (XV) above with an agent of formula NH₂CH(COL)CH(Z)OH, as typified in Preparation 30. Compounds of formula NH₂CH(COL)CH(Z)OH are available commercially, or can be made by methods disclosed in the chemical literature, or by routine modification thereof.

It will be apparent to those skilled in the art that other protection and subsequent deprotection regimes during synthesis of a compound of the invention may be achieved by conventional techniques, for example as described in the volumes by Greene and Wuts, and Kocienski, *supra*.

Where desired or necessary the compound of formula (I) is converted into a pharmaceutically acceptable salt thereof. A pharmaceutically acceptable salt of a compound of formula (I) may be conveniently be prepared by mixing together solutions of a compound of formula (I) and the desired acid or base, as appropriate. The salt may be precipitated from solution and collected by filtration, or may be collected by other means such as by evaporation of the solvent.

Certain compounds of the invention may be interconverted into certain other compounds of the invention by methods mentioned in the Examples and Preparations, and well-known methods from the literature.

Compounds of the invention are available by either the methods described herein in the Methods, Examples and Preparations or suitable adaptation thereof using methods known in the art. It is to be understood that the synthetic transformation methods mentioned herein may be carried out in various different sequences in order that the desired compounds can be efficiently assembled. The skilled chemist will exercise his judgement and skill as to the most efficient sequence of reactions for synthesis of a given target compound.

The compounds, salts, solvates (including hydrates) and prodrugs of the invention may be separated and purified by conventional methods.

Separation of diastereomers may be achieved by conventional techniques, e.g. by fractional crystallisation, chromatography or H.P.LC. of a stereoisomeric mixture of a compound of formula (I) or a suitable salt or derivative thereof. An individual enantiomer of a compound of formula (I) may also be prepared from a corresponding optically pure intermediate or by resolution, such as by H.P.L.C. of the corresponding racemate using a suitable chiral support or by fractional crystallisation of the diastereomeric salts formed by reaction of the corresponding racemate with a suitably optically active acid or base. In certain cases preferential crystallisation of one of the enantiomers can occur from a solution of a mixture of enantiomers, thus enriching the remaining solution in the other enantiomer.

For human use, the compounds of formula (I) or their salts can be administered alone, but will generally be administered in admixture with a pharmaceutically acceptable diluent or carrier selected with regard to the intended route of administration and standard pharmaceutical practice. For example, they can be administered orally, including sublingually, in the form of tablets containing such excipients as starch or lactose, or in capsules or ovules either alone or in admixture with excipients, or in the form of elixirs, solutions or suspensions containing flavouring or colouring agents. The compound or salt could be incorporated into capsules or tablets for targetting the colon or duodenum via delayed dissolution of said capsules or tablets for a particular time following oral administration. Dissolution could be controlled by susceptibility of the formulation to bacteria found in the duodenum or colon, so that no substantial dissolution takes places before reaching the target area of the gastrointestinal tract. The compounds or salts can be injected parenterally, for example, intravenously, intramuscularly, intradermally or subcutaneously. For parenteral administration, they are best used in the form of a sterile aqueous solution or suspension which may contain other substances, for example, enough salt or glucose to make the solution isotonic with blood. They can be administered topically, or transdermally, in the form of creams, gels, suspensions, lotions, ointments, dusting powders, sprays, foams, mousses, drug-incorporated dressings, solutions, sponges, fibres, microemulsions, films, ointments such as petrolatum or white soft paraffin based ointments or via a skin patch or other device. Penetration enhancers may be used, and the compound may be used in combination with cyclodextrins. In addition, the compound may be delivered using iontophoresis, electroporation, phonophoresis or sonophoresis. They could be administered directly onto a wound site. They could be incorporated into a coated suture. For example they can be incorporated into a lotion or cream consisting of an aqueous or oily emulsion of mineral oils; sorbitan monostearate; polysorbate 60; cetyl esters wax; cetearyl alcohol; 2-octyldodecanol; benzyl alcohol; water; polyethylene glycols and/or liquid paraffin, or they can be incorporated into a suitable ointment consisting of one or more of the following - mineral oil; liquid petrolatum; white petrolatum; propylene glycol; polyoxyethylene polyoxypropylene compound; emulsifying wax and water, or as hydrogel with cellulose or polyacrylate derivatives or other viscosity modifiers, or as a dry powder or liquid spray or aerosol with butane/propane, HFA, CFC, CO₂ or other suitable propellant, optionally also including a lubricant such as sorbitan trioleate, or as a drug-incorporated dressing either as a tulle dressing, with white soft paraffin or polyethylene glycols impregnated gauze dressings or with hydrogel, hydrocolloid, alginate or film dressings. The compound or salt could also be administered intraocularly for ophthalmic use e.g. via intraocular injection, or sustained release device, in a lens implant, via subconjunctival injection, or as an eye drop with appropriate buffers, viscosity modifiers (e.g. cellulose or polyacrylate derivatives), preservatives (e.g. benzalkonium chloride (BZK)) and agents to adjust tonicity (e.g. sodium chloride). Such formulation techniques are well-known in the art.

For certain uses, vaginal, rectal and nasal (e.g. by inhalation of a dry powder or aerosol) administration would be suitable.

All such formulations may also contain appropriate stabilisers and preservatives.

The compounds or their salts, solvates or prodrugs may be administered topically by the ocular route. They may be formulated as sterile, isotonic, pH adjusted, buffered suspensions or solutions. A polymer may be added such as crossed-linked polyacrylic acid, polyvinylalcohol, hyaluronic acid, a cellulosic polymer (e.g. hydroxypropylmethylcellulose, hydroxyethylcellulose, methyl cellulose), or a heteropolysaccharide polymer (e.g. gelan gum). Alternatively, they may be formulated in an ointment such as petrolatum or mineral oil, incorporated into bio-degradable (e.g. absorbable gel sponges, collagen) or non-biodegradable (e.g. silicone) implants, lenses or delivered via particulate or vesicular systems such as niosomes or liposomes. Formulations may be optionally combined with a preservative, such as benzalkonium chloride. In addition, they may be delivered using iontophoresis. The compound may also be used in combination with cyclodextrins.

An example of preferred formulation excipients of a compound, salt, solvate or prodrug according to the invention:

| Ingredients | % (w/w) composition |
|---|---|
| NaH₂PO₄ | 0.370 |
| Na₂HPO₄ | 0.567 |
| Glycine | 0.430 |
| Carbomer 940 | 1.000 |
| Water | to 100 |
| pH adjusted to -7 | |

The preferred formulation will be a buffered solution (preferably using monobasic and dibasic sodium phosphate) containing 0.5 to 5.0% of a cross-linked polyacrylic acid, pH adjusted to around 7 with the addition of a stabiliser such as glycine.

For oral and parenteral administration to human patients, the daily dosage level of the compounds of formula (I) or their salts will be from 0.001 to 20, preferably from 0.01 to 20, more preferably from 0.1 to 10, and most preferably from 0.5 to 5 mg/kg (in single or divided doses). Thus tablets or capsules of the compounds will contain from 0.1 to 500, preferably from 50 to 200, mg of active compound for administration singly or two or more at a time as appropriate.

For topical administration to human patients with acute/surgical wounds or scars, the daily dosage level of the compounds, in suspension or other formulation, could be from 0.01 to 50mg/ml, preferably from 0.3 to 30 mg/ml.

The dosage will vary with the size of the wound, whether or not the wound is open or closed or partially closed, and whether or not the skin is intact.

The physician in any event will determine the actual dosage which will be most suitable for a an individual patient and it will vary with the age, weight and response of the particular patient. The above dosages are exemplary of the average case; there can of course be individual instances where higher or lower dosage ranges are merited, and such are within the scope of this invention.

### Biological Test Methods

### PCP Inhibition

In order to determine potency of PCP inhibitors a fluorogenic PCP cleavage assay was used. This assay is based on the template of Beekman et al. (FEBS Letters (1996), 390: 221-225) using a fluorogenic substrate. The substrate (Dabcyl-Arg-Tyr-Tyr-Arg-Ala-Asp-Asp-Ala-Asn-Val-Glu(EDANS)-NH₂) contains the cleavage site of human PCP (Hojima et al., J Biol Chem (1985), 260: 15996-16003). Human PCP has been purified from supernatant of stable transfected CHO cells using hydrophobic interaction column followed by Superdex 200 gel filtration. 4 µg total protein of this enzyme preparation was incubated with various concentrations of the substance to be tested and 3x 10⁻⁶ M substrate in assay buffer (50 mM Tris-Base, pH 7.6 containing 150 mM NaCl, 5 mM CaCl₂, 1µM ZnCl₂ and 0.01 % Brij 35). The assay was performed in 96-well black fluorimeter plates and fluorescence was read continuously in a fluorimeter over 2.5 hours (λₑₓ = 340 nm, λₑₘ = 485 nm) at a constant 37°C with shaking. Release of the fluorogenic signal was in linear correlation to PCP activity. Reading of the mean velocity from 30 min after start of experiment until 2.5 hours was calculated by the Biolise software. IC₅₀ values were calculated by plotting % inhibition values against compound concentration using Tessela add in for Excel spreadsheet.

### MMP Inhibition

The ability of compounds to inhibit the cleavage of fluorogenic peptides by MMPs 1, 2, 9, and 14 is described below.

The assays for MMPs 2, 9, and 14 are based upon the original protocol described by Knight et al. (Fed.Euro.Biochem.Soc., 296 (3), 263-266; 1992) with the slight modifications given below.

### Inhibition of MMP-1

### (i) Enzyme Preparation

Catalytic domain MMP-1 was prepared at Pfizer Central Research. A stock solution of MMP-1 (1µM) was activated by the addition of aminophenylmercuric acetate (APMA), at a final concentration of 1mM, for 20 minutes at 37°C. MMP-1 was then diluted in Tris-HCl assay buffer (50mM Tris, 200mM NaCl, 5mM CaCl₂, 20µM ZnSO₄, 0.05% Brij 35) pH 7.5 to a concentration of 10nM. The final concentration of enzyme used in the assay was 1nM.

### (ii) Substrate

The fluorogenic substrate used in this assay was Dnp-Pro-β-cyclohexyl-Ala-Gly-Cys(Me)-His-Ala-Lys(N-Me-Ala)-NH₂ as originally described by Bickett et al (Anal. Biochem, 212, 58-64, 1993). The final substrate concentration used in the assay was 10µM.

### (iii) Determination of Enzyme Inhibition

Test compounds were dissolved in dimethyl sulphoxide and diluted with assay buffer so that no more than 1% dimethyl sulphoxide was present. Test compound and enzyme were added to each well of a 96 well plate and allowed to equilibrate for 15 minutes at 37°C in an orbital shaker prior to the addition of substrate. Plates were then incubated for 1 hour at 37°C prior to determination of fluorescence (substrate cleavage) using a fluorimeter (Fluostar; BMG LabTechnologies, Aylesbury, UK) at an excitation wavelength of 355nm and emission wavelength of 440nm. The potency of inhibitors was measured from the amount of substrate cleavage obtained using a range of test compound concentrations, and, from the resulting dose-response curve, an IC₅₀ value (the concentration of inhibitor required to inhibit 50% of the enzyme activity) was calculated.

### Inhibition of MMP-2 and MMP-9

### (i) Enzyme Preparation

Catalytic domain MMP-2 and MMP-9 were prepared at Pfizer Central Research. A stock solution of MMP-2 / MMP-9 (1µM) was activated by the addition of aminophenylmercuric acetate (APMA). For MMP-2 and MMP-9, a final concentration of 1 mM APMA was added, followed by incubation for 1 hour at 37°C. The enzymes were then diluted in Tris-HCl assay buffer (100mM Tris, 100mM NaCl, 10mM CaCl₂ and 0.16% Brij 35, pH 7.5), to a concentration of 10nM. The final concentration of enzyme used in the assays was 1 nM.

### (ii) Substrate

The fluorogenic substrate used in this screen was Mca-Arg-Pro-Lys-Pro-Tyr-Ala-Nva-Trp-Met-Lys(Dnp)-NH₂ (Bachem Ltd, Essex, UK) as originally described by Nagase et al (J.Biol.Chem., 269(33), 20952-20957, 1994). This substrate was selected because it has a balanced hydrolysis rate against MMPs 2 and 9 (k_{cat} / kₘ of 54,000 and 55,300 s⁻¹ M⁻¹ respectively). The final substrate concentration used in the assay was 5µM.

### (iii) Determination of Enzyme Inhibition

Test compounds were dissolved in dimethyl sulphoxide and diluted with test buffer solution (as above) so that no more than 1% dimethyl sulphoxide was present. Test compound and enzyme were added to each well of a 96 well plate and allowed to equilibrate for 15 minutes at 37°C in an orbital shaker prior to the addition of substrate. Plates were then incubated for 1 hour at 37°C prior to determination of fluorescence using a fluorimeter (Fluostar, BMG LabTechnologies, Aylesbury, UK) at an excitation wavelength of 328nm and emission wavelength of 393nm. The potency of inhibitors was measured from the amount of substrate cleavage obtained using a range of test compound concentrations, and, from the resulting dose-response curve, an IC₅₀ value (the concentration of inhibitor required to inhibit 50% of the enzyme activity) was calculated.

### Inhibition of MMP-14

### (i) Enzyme Preparation

Catalytic domain MMP-14 was purchased from Prof. Tschesche, Department of Biochemistry, Faculty of Chemistry, University of Bielefeld, Germany. A 10µM enzyme stock solution was activated for 20 minutes at 25°C following the addition of 5µg/ml of trypsin (Sigma, Dorset, UK). The trypsin activity was then neutralised by the addition of 50µg/ml of soyabean trypsin inhibitor (Sigma, Dorset, UK), prior to dilution of this enzyme stock solution in Tris-HCl assay buffer (100mM Tris, 100mM NaCl, 10mM CaCl₂ and 0.16% Brij 35, pH 7.5) to a concentration of 10nM. The final concentration of enzyme used in the assay was 1 nM.

### (ii) Substrate

The fluorogenic substrate used in this screen was Mca-Pro-Leu-Gly-Leu-Dpa-Ala-Arg-NH₂ (Bachem Ltd, Essex, UK) as described by Will et al (J. Biol.Chem., 271(29), 17119-17123, 1996). The final substrate concentration used in the assay was 10µM.

Determination of enzyme inhibition by test compounds was performed in the same manner as described for MMPs-2 and -9 above.

The compounds of Examples 1-12 had PCP IC₅₀ values of 100µM and below.

All references mentioned herein in this text are incorporated by reference in their entirety.

### EXAMPLES AND PREPARATIONS:

Melting points were determined using open glass capillary tubes and a Gallenkamp melting point apparatus and are uncorrected. Nuclear magnetic resonance (NMR) data were obtained using Varian Unity Inova-400, Varian Unity Inova-300 or Bruker AC300 spectrometers and are quoted in parts per million from tetramethylsilane. Mass spectral (MS) data were obtained on a Finnigan Mat. TSQ 7000 or a Fisons Instruments Trio 1000. The calculated and observed ions quoted refer to the isotopic composition of lowest mass. Infra red (IR) spectra were measured using a Nicolet Magna 550 Fourier transform infra-red spectrometer. Flash chromatography refers to column chromatography on silica gel (Kieselgel 60, 230-400 mesh, from E. Merck, Darmstadt. Kieselgel 60 F₂₅₄ plates from E. Merck were used for TLC, and compounds were visualised using UV light, 5% aqueous potassium permanganate or Dragendorff's reagent (oversprayed with aqueous sodium nitrite). Thermal analyses by Differential Scanning Calorimetry (DSC) and ThermoGravimetric Analysis (TGA) were obtained using Perkin Elmer DSC7 and TGA7. Moisture sorption characteristics were recorded using Surface Measurement Systems Ltd. Automated Water Sorption Analyser DVS 1. Water content was determined on a Mitsubishi CA100 (Coulometric Karl Fisher Titrator). Powder X-ray diffraction (PXRD) pattern was determined using a Siemens D5000 powder X-ray diffractometer fitted with an automatic sample changer, a theta-theta goniometer, automatic beam divergence slits, a secondary monochromator and a scintillation counter. Other measurements were taken using standard equipment. Hexane refers to a mixture of hexanes (hplc grade) b.p. 65-70°C. Ether refers to diethyl ether. Acetic acid refers to glacial acetic acid. 1-Hydroxy-7-aza-1H-1,2,3-benzotriazole (HOAt), N-[(dimethylamino)-1H-1,2,3-triazolo[4,5-b]pyridin-1-ylmethylene]-N-methylmethaninium hexafluorophosphate N-oxide (HATU) and 7-azabenzotriazol-1-yloxytris(pyrrolidino)phosphonium hexafluorophosphate (PyAOP) were purchased from PerSeptive Biosystems U.K. Ltd. "DIPE" refers to diisopropyl ether. Reverse-phase silica gel for flash chromatography was obtained from Fluka (Fluka 100, C₁₈, 40-63µ). Pentane refers to High Performance Liquid Chromatography (HPLC) grade n-pentane (b.pt.35-37°C). Nomenclature has been allocated using a program available from IUPAC. Standard abbreviations are used throughout, e.g. "Me" is methyl, "Et" is ethyl, "Pr" is propyl, "Ph" is phenyl, etc. It was noticed that during certain repetitions of the methods disclosed in the Examples and Preparations that some racemisation appeared to have taken place. It was found in some cases that specific desired enantiomers can be separated from mixtures thereof by routine methods such as by differential crystallisation.
^{a}HPLC autopurification performed using 2 columns - Phenomenex LUNA C8 150x21.2mm, 10µm and Phenomenex MAGELLEN C18 150x21.2mm, 5µm, eluting with a gradient system of organic solvent [ammonium acetate (aq) 100mM : acetonitrile (1 : 9)]: aqueous solvent [ammonium acetate (aq) 100mM : acetonitrile (9 : 1)]
^{b}HPLC autopurification performed using 2 columns - Phenomenex LUNA C8 150x21.2mm, 10µm and Phenomenex MAGELLEN C18 150x21.2mm, 5µm, eluting with a gradient system of organic solvent (acetonitrile) : aqueous solvent (0.1% aqueous trifluoroacetic acid)

### Example 1

### (3R)-6-Cyclohexyl-N-hydroxy-3-(3-{[(phenylsulfonyl)amino]carbonyl}-1,2,4-oxadiazol-5-yl)hexanamide

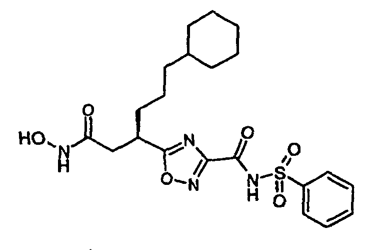

To a solution of the title compound from Preparation 2 (2.24 g, 5.0 mmol) in THF (40 mL) was added 2,6-lutidine (1.07 g, 10.0 mmol), the solution was cooled to 0 °C and then iso-butylchloroformate (0.66 mL, 5.05 mmol) was added. The reaction was stirred at 0 °C for 1 hour then O-trimethylsilyl hydroxylamine (1.83 mL, 15.0 mmol) was added, the reaction was warmed to room temperature and stirred overnight. Methanol (25 mL) was added, and stirring was continued for 30 minutes. The solvent was removed in vacuo and the residue was then partitioned between ethyl acetate (75 mL) and 2M aqueous hydrochloric acid (35 mL). The layers were separated and the aqueous layer was extracted with ethyl acetate (60 mL). The combined organic extracts were dried (Na₂SO₄) and the solvent was removed in vacuo. The residue was dissolved in water/acetonitrile and purified by chromatography on a Biotage CP-18 reverse phase 40S cartridge (graded elution of acetonitrile/water 30:70 to 50:50) to give a white foam which was recrystallised from toluene to give the title compound as a white crystalline solid (1.0 g).
Mpt 80-90 °C.
¹H NMR (400MHz, D₆-DMSO) □□0.74-0.89 (m, 2H), 1.06-1.24 (m, 8H), 1.53-1.69 (m, 7H), 2.66-2.81 (m, 2H), 3.39-3.47 (m, 1H), 7.52-7.69 (m, 3H), 7.94 (d, 2H), 8.05 (s, 1H), 10.9-11.4 (br s, 2H).
LRMS (ES) 487 (M+Na).
Anal. Calcd. For C₂₁H₂₈N₄O₆S + 0.15 CH₂Cl₂ + 0.2 toluene: calc C = 50.94, H = 6.43, N = 10.54, found C = 51.04, H = 6.05, N = 10.45.

### Example 2

### (3R)-6-Cyclohexyl-N-hydroxy-3-[3-({[(4-methylphenyl)sulfonyl]amino}carbonyl)-1,2,4-oxadiazol-5-yl]hexanamide

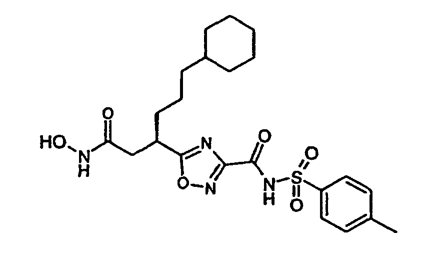

To a solution of the title compound from Preparation 4 (0.23 g, 0.5 mmol) in THF (12 mL) was added triethylamine (0.14 mL, 1.0 mmol). The reaction was cooled to 0 °C in an ice bath, then iso-butylchloroformate (0.06 mL, 0.5 mmol) was added and a precipitate began to form immediately. The mixture was stirred for 1 hour, then O-trimethylsilyl hydroxylamine (0.20 mL, 1.6 mmol) was added and the reaction was warmed to room temperature and stirred overnight. Methanol (10 mL) was added, stirring was continued for 1.5 hours, and then the solvent was removed in vacuo. The residue was dissolved in ethyl acetate (50 mL) and washed with water (50 mL). The organic layer was dried (MgSO₄) and the solvent was evaporated in vacuo. The residue was purified by flash chromatography on silica gel (graded elution with dichloromethane/methanol/glacial acetic acid 99:1:0.1 to 98:2:0.2 to 95:5:0.5 to 90:10:1) to give the title compound as an orange foam which was azeotroped with toluene to remove traces of acetic acid (0.067 g).
Mpt 82-84 °C.
¹H NMR (400 MHz, D₆-DMSO) □□0.72-0.84 (m, 2H), 1.05-1.20 (m, 8H), 1.53-1.75 (m, 7H), 2.36 (s, 3H), 2.38-2.53 (m, 2H), 3.40-3.49 (m, 1 H), 7.37 (d, 2H), 7.71 (d, 2H), 10.37 (s, 1H).
LRMS (ES) 477 (M-H).
Anal. Calcd. For C₂₂H₃₀N₄O₆S + 0.2 CH₂Cl₂ + 0.1 PhMe: C, 54.49; H, 6.23; N, 11.10. Found C, 54.35; H, 6.37; N, 10.95.

### Example 3

### (3R)-6-Cyclohexyl-N-hydroxy-3-[3-({[(4-methoxyphenyl)sulfonyl]amino}carbonyl)-1,2,4-oxadiazol-5-yl]hexanamide

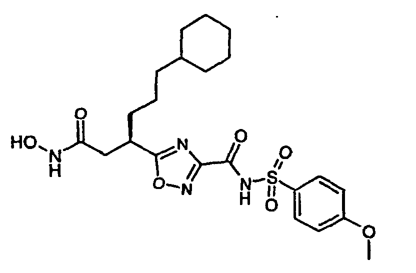

To a solution of the title compound from Preparation 6 (0.25 g, 0.5 mmol) in THF (5 mL) was added 1,1-carbonyldiimidazole (0.093 g, 0.6 mmol). The mixture was stirred for 1 hour, then O-trimethylsilyl hydroxylamine (0.19 mL, 1.6 mmol) was added and the reaction was stirred at room temperature overnight. Methanol (10 mL) was added, stirring was continued for 1 hour and then the solvent was removed in vacuo. The residue was purified by flash chromatography on a Biotage 401 flash system (dichloromethane/methanol/glacial acetic acid 90:10:1.0 as eluant) to give an orange foam which was triturated with diisopropyl ether. The solid was collected by filtration and dried to give the title compound as an orange solid (0.35 g).
Mpt 55-62 °C.
¹H NMR (400 MHz, D₆-DMSO) □□0.73-0.82 (m, 2H), 1.00-1.20 (m, 8H), 1.50-1.77 (m, 7H), 2.38-2.53 (m, 2H), 3.37-3.49 (m, 1H), 3.80 (s, 3H), 7.07 (d, 2H), 7.86 (d, 2H), 10.40 (s, 1H).
LRMS (ES) 493 (M-H).
Anal. Calcd. For C₂₂H₃₀N₄O₆S + 0.4 H₂O: C, 52.66; H, 6.19; N, 11.17. Found C, 52.77; H, 6.26; N, 11.09.

### Example 4

### (3R)-6-Cyclohexyl-3-[3-({[(4-fluorophenyl)sulfonyl]amino}carbonyl)-1,2,4-oxadiazol-5-yl]-N-hydroxyhexanamide

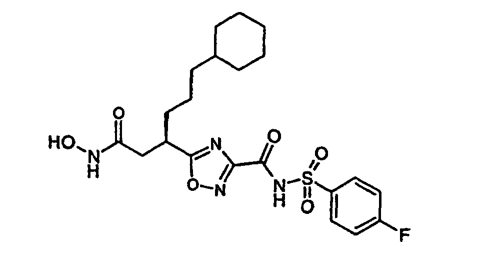

The title compound was obtained as a white foam from the title compound from Preparation 8, using a similar method to that described in Example 2.
¹H NMR (400 MHz, D₆-DMSO) □□0.72-0.84 (m, 2H), 1.03-1.20 (m, 8H), 1.52-1.65 (m, 7H), 2.38-2.52 (m, 2H), 3.36-3.47 (m, 1 H), 7.30 (m, 2H), 7.92 (m, 2H), 10.37 (s, 1H).
LRMS (ES) 481 (M-H).
Anal. Calcd. For C₂₁H₂₇FN₄O₆S + 0.7 H₂O: C, 50.94; H, 5.78; N, 11.32. Found C, 50.91; H, 5.69; N, 11.32.

### Example 5

### (3R)-6-Cyclohexyl-N-hydroxy-3-[3-({[(4-isopropylphenyl)sulfonyl]amino} carbonyl)-1,2,4-oxadiazol-5-yl]hexanamide

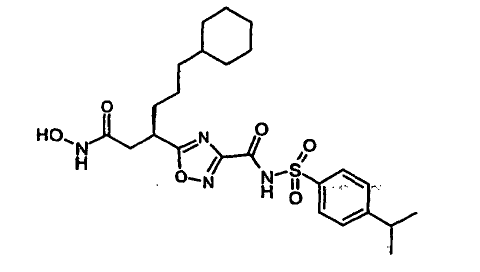

The title compound was obtained as a white foam from the title compound from Preparation 10, using a similar method to that described in Example 2.
¹H NMR (400 MHz, D₆-DMSO) □□0.72-0.83 (m, 2H), 1.02-1.24 (m, 8H), 1.21 (d, 6H), 1.52-1.65 (m, 7H), 2.39-2.52 (m, 2H), 2.94-3.01 (m, 1 H), 3.41-3.50 (m, 1H), 7.47 (d, 2H), 7.78 (d, 2H), 10.37 (s, 1H).
LRMS (ES) 505 (M-H).
Anal. Calcd. For C₂₄H₃₄N₄O₆S + 0.4 H₂O: C, 56.10; H, 6.83; N, 10.90. Found C, 56.28; H, 6.91; N, 10.70.

### Example 6

### (3R)-6-Cyclohexyl-3-[3-({[(3,4-dimethoxyphenyl)sulfonyl]amino}carbonyl)-1,2,4-oxadiazol-5-yl]-N-hydroxyhexanamide

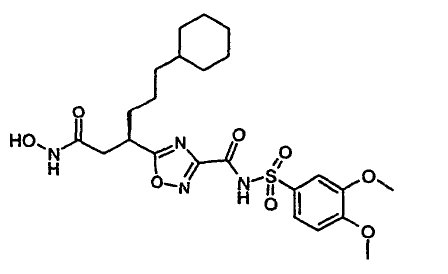

The title compound was obtained as an orange foam from the title compound from Preparation 12, using a similar method to that described in Example 3.
¹H NMR (400 MHz, D₆-DMSO) □□0.71-0.85 (m, 2H), 1.04-1.22 (m, 8H), 1.52-1.67 (m, 7H), 2.40-2.53 (m, 2H), 3.41-3.49 (m, 1 H), 3.80 (s, 3H), 3.82 (s, 3H), 7.13 (d, 1 H), 7.46 (s, 1H), 7.54 (d, 1 H), 10.36 (s, 1H).
LRMS (ES) 523 (M-H).
Anal. Calcd. For C₂₃H₃₂N₄O₈S: C, 52.66; H, 6.15; N, 10.68. Found C, 52.31; H, 6.30; N, 10.39.

### Example 7

### (3R)-6-Cyclohexyl-N-hydroxy-3-(3-{[(8-quinolinylsulfonyl)amino]carbonyl}-1,2,4-oxadiazol-5-yl)hexanamide

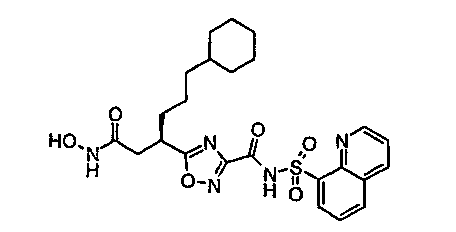

The title compound was obtained as a white solid from the title compound from Preparation 14, using a similar method to that described in Example 1.
Mpt 155-158 °C.
¹H NMR (400 MHz, D₆-DMSO) □□0.72-0.86 (m, 2H), 1.02-1.22 (m, 8H), 1.50-1.68 (m, 7H), 2.34-2.53 (m, 2H), 3.33-3.44 (m, 1 H), 7.53 (dd, 1 H), 7.66 (dd, 1H), 8.07 (d, 1 H), 8.32-8.41 (m, 2H), 8.63 (s, 1 H), 8.88-8.94 (m, 1H), 10.36 (s, 1H).
LRMS (ES) 514 (M-H).
Anal. Calcd. For C₂₄H₂₉N₅O₆S + 1.3 H₂O: C, 53.48; H, 5.91; N, 12.99. Found C, 53.10; H, 5.72; N, 12.80.

### Example 8

### (3R)-6-Cyclohexyl-3-[3-({[(3,5-dimethyl-4-isoxazolyl)sulfonyl]amino}carbonyl)-1,2,4-oxadiazol-5-yl]-N-hydroxyhexanamide

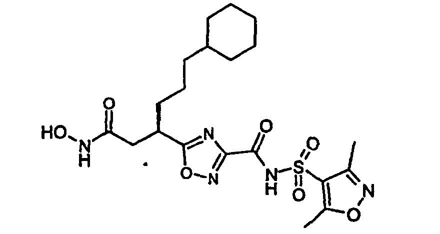

The title compound was obtained as an orange solid from the title compound from Preparation 16, using a similar method to that described in Example 1.
Mpt 122-124 °C.
¹H NMR (400 MHz, D₆-DMSO) □□0.73-0.85 (m, 2H), 1.03-1.22 (m, 8H), 1.51-1.68 (m, 7H), 2.26 (s, 3H), 2.38-2.50 (m, 2H), 2.52 (s, 3H), 3.34-3.44 (m, 1 H), 9.63 (s, 1H), 10.35 (s, 1H).
LRMS (ES) 482 (M-H).
Anal. Calcd. For C₂₀H₂₉N₅O₇S + 1.6 H₂O: C, 46.88; H, 6.33; N, 13.39. Found C, 46.57 H, 5.89; N, 13.33.

### Example 9

### (3R)-6-Cyclohexyl-N-hydroxy-3-(3-{[(isopropylsulfonyl)amino]carbonyl}-1,2,4-oxadiazol-5-yl)hexanamide

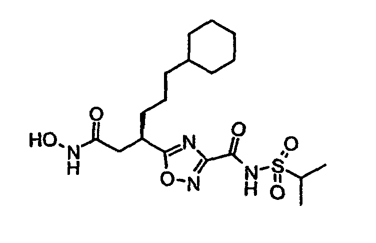

The title compound was obtained as an orange solid from the title compound from Preparation 18, using a similar method to that described in Example 3, apart from the final compound was azeotroped with toluene to remove traces of acetic acid.
¹H NMR (400 MHz, D₆-DMSO) □□0.73-0.87 (m, 2H), 1.03-1.23 (m, 14H), 1.51-1.67 (m, 7H), 2.38-2.50 (m, 2H), 3.32-3.48 (m, 2H), 8.62 (s, 1H), 10.38 (s, 1H).
LRMS (ES) 429 (M-H).
Anal. Calcd. For C₁₈H₃₀N₄O₆S + 0.08 H₂O + 1.28 CH₂Cl₂: C, 42.83; H, 6.10; N, 10.36. Found C, 42.44; H, 5.79; N, 10.76.

### Example 10

### (3R)-6-Cyclohexyl-N-hydroxy-3-(3-{[methyl(methylsulfonyl)amino]carbonyl}-1,2,4-oxadiazol-5-yl]hexanamide

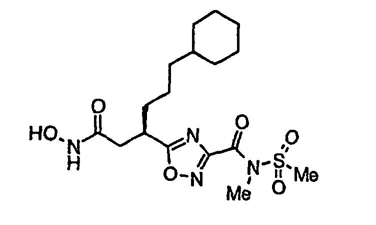

To a solution of the title compound from Preparation 20 (0.22 g, 0.54 mmol) in tetrahydrofuran (6 mL) and diethyl ether (6 mL) at 0 °C was added N-methylmorpholine (0.065 mL, 0.59 mmol) followed by ethyl chloroformate (0.057 mL, 0.59 mmol). The reaction was stirred for 1 hour then aqueous hydroxylamine (0.076 mL of a 50% w/v solution, 1.3 mmol) was added. The reaction was slowly warmed to room temperature over 2 hours and then the solvents were removed in vacuo. The residue was taken up in ethyl acetate (10 mL) and washed with 6M aqueous hydrochloric acid solution (10 mL), water (10 mL) then saturated aqueous sodium chloride solution (10 mL). The organic layer was dried (Na₂SO₄) and the solvent removed in vacuo. The residue was purified by flash chromatography on silica gel (graded elution of dichloromethane/methanol/acetic acid 195:5:1 to 190:10:1), and the azeotroped with toluene to remove traces of acetic acid to give the title compound as a white foam (0.050 g).
¹H NMR (400 MHz, D₆-DMSO) □□0.73-0.86 (m, 2H), 1.02-1.23 (m, 8H), 1.53-1.74 (m, 7H), 2.38-2.50 (m, 2H), 3.27 (s, 3H), 3.46-3.58 (m, 4H), 8.63 (s, 1 H), 10.39 (s, 1H).
LRMS (ES) 439 (M+Na).
Anal. Calcd. For C₁₇H₂₈N₄O₆S + 0.09 CH₂Cl₂ + 0.09 PhMe: C, 49.22; H, 6.74; N, 12.96. Found C, 49.17 H, 6.85; N, 12.82.

### Example 11

### (3R)-6-Cyclohexyl-N-hydroxy-3-(3-{[(phenylmethyl)sulfonyl]amino}carbonyl)-1,2,4-oxadiazol-5-yl)hexanamide

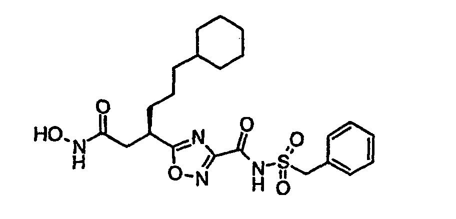

The title compound was obtained as an orange solid from the title compound from Preparation 22, using a similar method to that described in Example 3, apart from the final compound was purified by flash chromatography on silica gel (elution with dichloromethane/methanol/acetic acid 188:12:1) and then azeotroped with toluene to remove traces of acetic acid.
¹H NMR (400 MHz, CDCl₃) □□0.73-0.87 (m, 2H), 1.01-1.22 (m, 8H), 1.51-1.77 (m, 7H), 2.38-2.50 (m, 2H), 3.40 (m, 1H), 4.34 (s, 2H), 7.17-7.33 (m, 5H), 8.64 (s, 1 H), 10.37 (s, 1H).
LRMS (ES) 477 (M-H).
HRMS (ES) 501 (M+Na). Calcd. For C₂₂H₃₀N₄O₆S + Na: 501.1778. Found 501.1767.

### Example 12

### (3R)-6-Cyclohexyl-N-hydroxy-3-(3-{[(3-pyridylsulfonyl)amino]carbonyl)-1,2,4-oxadiazol-5-yl)hexanamide

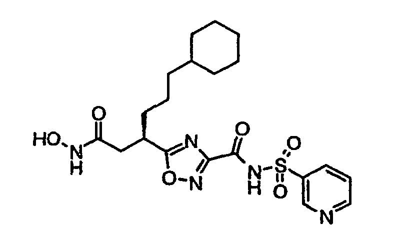

The title compound was obtained as an orange solid from the title compound from Preparation 24, using a similar method to that described in Example 3, apart from the final compound was purified by flash chromatography on silica gel (graded elution of dichloromethane/methanol/acetic acid 195:5:1 to 190:10:1) and then azeotroped with toluene to remove traces of acetic acid.
¹H NMR (400 MHz, D₆-DMSO) □□0.70-0.85 (m, 2H), 1.01-1.24 (m, 8H), 1.48-1.78 (m, 7H), 2.38-2.50 (m, 2H), 3.39 (m, 1H), 7.49 (m, 1 H), 8.10 (d, 1H), 8.62 (d, 1 H), 8.96 (s, 1H), 10.36 (s, 1H).
LRMS (ES) 464 (M-H).

### Preparation 1

### tert-Butyl (3R)-6-cyclohexyl-3-(3-{[(phenylsulfonyl)amino]carbonyl}-1,2,4-oxadiazol-5-yl)hexanoate

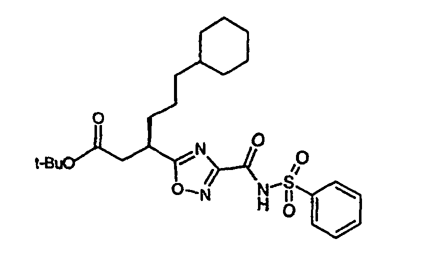

To a suspension of sodium hydride (0.65 g of 60% dispersion in mineral oil, 16.4 mmol) in tetrahydrofuran (80 mL) at 0 °C was added a solution of the title compound from Preparation 28 (3.0 g, 8.2 mmol) and freshly distilled benzenesulfonyl chloride (1.15 mL, 9.0 mmol) in tetrahydrofuran (20 mL) dropwise. The reaction was stirred at 0 °C for 3 hours then quenched with 2M aqueous hydrochloric acid and warmed to room temperature. The mixture was diluted with water (50 mL) and then extracted with ethyl acetate (3 x 100 mL). The combined organic extracts were dried (Na₂SO₄) and the solvent was removed in vacuo. The residue was purified by flash chromatography on a Biotage 405 cartridge (graded elution of dichloromethane to dichloromethane /methanol 95:5) to give the title compound as a clear oil (2.8 g).
¹H NMR (400 MHz, CDCl₃) □ 0.78-0.91 (m, 2H), 1.11-1.55 (m, 18H), 1.55-1.79 (m, 6H), 2.65 (dd, 1H), 2.80 (dd, 1H), 3.44-3.52 (m, 1H), 7.56 (dd, 2H), 7.66 (dd, 1H), 8.17 (d, 2H).
LRMS (ES) 504 (M-H).
Anal. Calcd. For C₂₅H₃₅N₃O₆S + 0.5 H₂O: C, 58.35; H, 7.05; N, 8.16. Found C, 58.49; H, 6.93; N, 8.16.

### Preparation 2

### (3R)-6-Cyclohexyl-3-(3-{[(phenylsulfonyl)amino]carbonyl}-1,2,4-oxadiazol-5-yl)hexanoic acid

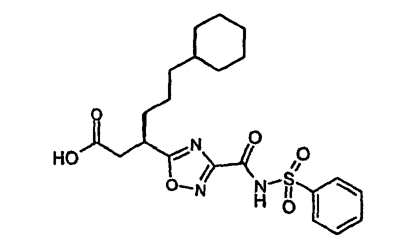

The title compound from Preparation 1 (2.65 g, 1.0 mmol) was dissolved in dichloromethane (30 mL) then trifluoroacetic acid (6 mL) was added and the reaction was stirred at room temperature for 2 hours. A further portion of trifluoroacetic acid (1 mL) was added and stirring was continued for 1 hour. The solvent was removed in vacuo, and the residue was azeotroped with toluene (3 times) to remove traces of trifluoroacetic acid, to give the title compound as a viscous oil (2.28 g).
¹H NMR (400 MHz, D₆-DMSO) □□0.72-0.85 (m, 2H), 1.01-1.26 (m, 8H), 1.52-1.69 (m, 7H), 2.67-2.81 (m, 2H), 3.39-3.50 (m, 1H), 7.56-7.68 (m, 2H), 7.68-7.76 (m, 1H), 7.97 (d, 2H).
LRMS (ES) 448 (M-H).

### Preparation 3

### tert-Butyl (3R)-6-cyclohexyl-3-[3-({[(4-methylphenyl)sulfonyl]amino}carbonyl)-1,2,4-oxadiazol-5-yl]hexanoate

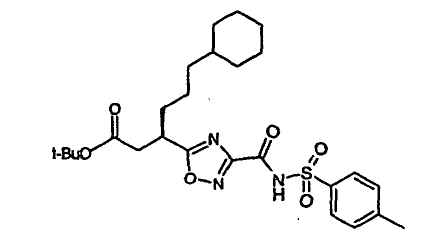

To a suspension of sodium hydride (0.19 g of 60% dispersion in mineral oil, 4.8 mmol) in tetrahydrofuran (30 mL) at 0 °C was added a solution of the title compound from Preparation 28 (0.80 g, 2.2 mmol) and p-toluenesulfonyl chloride (0.46 g, 2.4 mmol) in tetrahydrofuran (20 mL) dropwise. The reaction was stirred at 0 °C for 1 hour then warmed to room temperature and stirred overnight. Saturated aqueous ammonium chloride solution was added, the pH was adjusted to 3 with 2M aqueous hydrochloric acid solution and then extracted with dichloromethane (3 x 50 mL). The combined organic extracts were dried (MgSO₄) and the solvent was removed in vacuo. The residue was purified by flash chromatography on silica gel (graded elution of pentane/ dichloromethane 3:1 to 2:1 to 1:1 to 100% dichloromethane to dichloromethane /methanol 98:2) to give the title compound as a clear oil (0.40 g).
¹H NMR (400 MHz, D₆-DMSO) □□0.71-0.84 (m, 2H), 1.03-1.25 (m, 8H), 1.27 (s, 9H), 1.53-1.66 (m, 7H), 2.38 (s, 3H), 2.64-2.72 (m, 2H), 3.39-3.46 (m, 1H), 7.42 (d, 2H), 7.85 (d, 2H).
LRMS (ES) 518 (M-H).
Anal. Calcd. For C₂₆H₃₇N₃O₆S + 0.6 H₂O: C, 58.87; H, 7.26; N, 7.92. Found C, 58.66; H, 7.01; N, 7.70.

### Preparation 4

### (3R)-6-Cyclohexyl-3-[3-({[(4-methylphenyl)sulfonyl]amino}carbonyl)-1,2,4-oxadiazol-5-yl]hexanoic acid

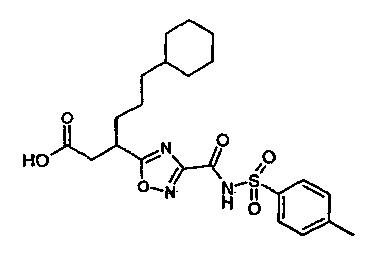

Hydrogen chloride gas was bubbled through dichloromethane (10 mL) until the solution was saturated, then the title compound from Preparation 3 (0.40 g, 0.8 mmol) was added and the reaction was stirred at room temperature overnight. More hydrogen chloride gas was bubbled through the reaction mixture, then stirring was continued for another 4 hours. The reaction was not complete, so trifluoroacetic acid (1.0 mL) was added and the reaction was stirred overnight The solvent was removed in vacuo and azeotroped with toluene, to give the title compound as a clear oil (0.24 g).
¹H NMR (400 MHz, D₆-DMSO) □□0.72-0.85 (m, 2H), 1.01-1.25 (m, 8H), 1.51-1.70 (m, 7H), 2.37 (s, 3H), 2.65-2.72 (m, 2H), 3.36-3.48 (m, 1H), 7.40 (d, 2H), 7.85 (d, 2H).
LRMS (ES) 462 (M-H).
Anal. Calcd. For C₂₂H₂₉N₃O₆S + 0.2 DIPE + 0.3 H₂O: C, 56.94; H, 6.67; N, 8.59. Found C, 56.91; H, 6.64; N, 8.39.

### Preparation 5

### tert-Butyl (3R)-6-cyclohexyl-3-[3-({[(4-methoxyphenyl)sulfonyl]amino}carbonyl)-1,2,4-oxadiazol-5-yl]hexanoate

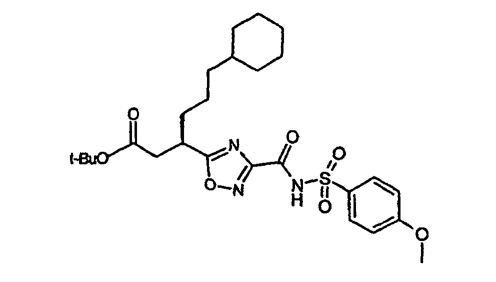

The title compound was obtained as a white foam from the title compound from Preparation 28 and 4-methoxybenzenesulfonyl chloride, using a similar method to that described in Preparation 3.
¹H NMR (400 MHz, D₆-DMSO) □□0.71-0.84 (m, 2H), 1.03-1.25 (m, 8H), 1.27 (s, 9H), 1.52-1.69 (m, 7H), 2.67-2.74 (m, 2H), 3.38-3.48 (m, 1H), 3.84 (s, 3H), 7.13 (d, 2H), 7.90 (d, 2H).
LRMS (ES) 534 (M-H).
Anal. Calcd. For C₂₆H₃₇N₃O₇S: C, 58.30; H, 6.96; N, 7.84. Found C, 58.20; H, 7.02; N, 7.76.

### Preparation 6

### (3R)-6-Cyclohexyl-3-[3-({[(4-methoxyphenyl)sulfonyl]amino}carbonyl)-1,2,4-oxadiazol-5-yl]hexanoic acid

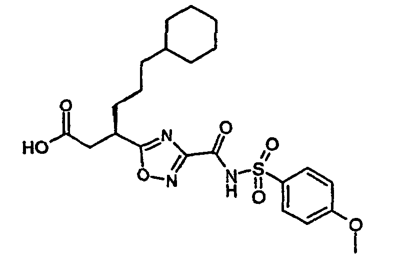

The title compound from Preparation 5 (0.55 g, 1.0 mmol) was dissolved in dichloromethane, then trifluoroacetic acid (1 mL) was added and the reaction was stirred at room temperature overnight. A further 1 mL of trifluoroacetic acid was added and stirring was continued for 4 hours. The solvent was then removed in vacuo and azeotroped with toluene to give the title compound as a white solid (0.50 g).
Mpt 123-127 °C.
¹H NMR (400 MHz, D₆-DMSO) □□0.73-0.85 (m, 2H), 1.03-1.24 (m, 8H), 1.52-1.68 (m, 7H), 2.67-2.80 (m, 2H), 3.38-3.48 (m, 1H), 3.84 (s, 3H), 7.12 (d, 2H), 7.91 (d, 2H).
LRMS (ES) 478 (M-H).
Anal. Calcd. For C₂₂H₂₉N₃O₇S: C, 55.10; H, 6.10; N, 8.76. Found C, 55.29; H, 6.07; N, 8.71.

### Preparation 7

### tert-Butyl (3R)-6-cyclohexyl-3-[3-({[(4-fluorophenyl)sulfonyl]amino}carbonyl)-1,2,4-oxadiazol-5-yl]hexanoate

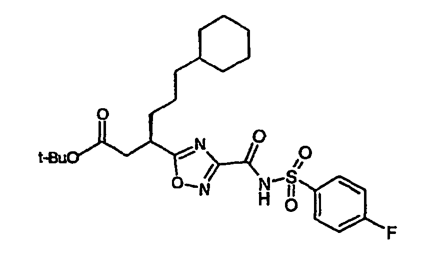

The title compound was obtained as a white foam from the title compound from Preparation 28 and 4-fluorobenzenesulfonyl chloride, using a similar method to that described in Preparation 1.
¹H NMR (400 MHz, D₆-DMSO) □□0.71-0.84 (m, 2H), 1.03-1.32 (m, 17H), 1.52-1.69 (m, 7H), 2.67-2.77 (m, 2H), 3.38-3.46 (m, 1H), 7.42 (m, 2H), 8.03 (m, 2H).
LRMS (ES) 522 (M-H).
Anal. Calcd. For C₂₅H₃₄FN₃O₆S + 0.25 CH₂Cl₂: C, 55.66; H, 6.38; N, 7.71. Found C, 55.72; H, 6.50; N, 7.95.

### Preparation 8

### (3R)-6-Cyclohexyl-3-[3-({[(4-fluorophenyl)sulfonyl]amino}carbonyl)-1,2,4-oxadiazol-5-yl]hexanoic acid

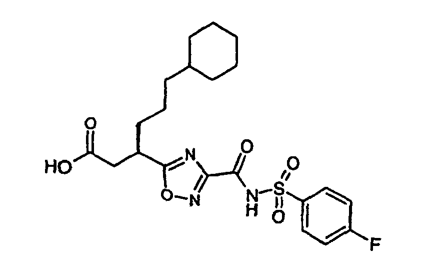

The title compound was obtained as a white foam from the title compound from Preparation 7, using a similar method to that described in Preparation 2, apart from the reaction was carried out in toluene.
¹H NMR (400 MHz, D₆-DMSO) □□0.73-0.87 (m, 2H), 1.03-1.24 (m, 8H), 1.53-1.69 (m, 7H), 2.67-2.80 (m, 2H), 3.37-3.46 (m, 1H), 7.35 (m, 2H), 7.99 (m, 2H).
LRMS (ES) 466 (M-H).
Anal. Calcd. For C₂₁H₂₆FN₃O₆S + 0.05 PhMe: C, 54.32; H, 5.64; N, 8.90. Found C, 53.95; H, 6.03; N, 8.54.

### Preparation 9

### tert-Butyl (3R)-6-cyclohexyl-3-[3-({[(4-isopropylphenyl)sulfonyl]amino}carbonyl)-1,2,4-oxadiazol-5-yl]hexanoate

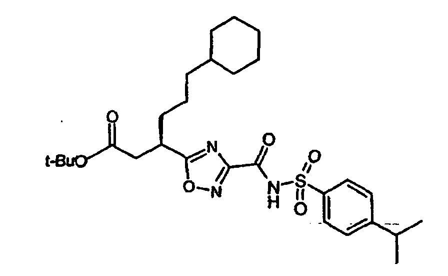

The title compound was obtained as a white foam from the title compound from Preparation 28 and 4-isopropylbenzenesulfonyl chloride, using a similar method to that described in Preparation 1.
¹H NMR (400 MHz, D₆-DMSO) □□0.72-0.87 (m, 2H), 1.03-1.28 (m, 8H), 1.21 (d, 6H), 1.27 (s, 9H), 1.51-1.68 (m, 7H), 2.68-2.73 (m, 2H), 2.92-3.04 (m, 1H), 3.38-3.47 (m, 1H), 7.48 (d, 2H), 7.89 (d, 2H).
Anal. Calcd. For C₂₈H₄₁N₃O₆S + 0.02 CH₂Cl₂: C, 61.26; H, 7.53; N, 7.65. Found C, 60.86; H, 7.64; N, 7.69.

### Preparation 10

### (3R)-6-Cyclohexyl-3-[3-({[(4-isopropylphenyl)sulfonyl]amino}carbonyl)-1,2,4-oxadiazol-5-yl]hexanoic acid

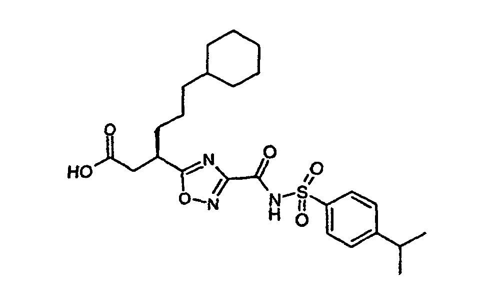

The title compound was obtained as a white foam from the title compound from Preparation 9, using a similar method to that described in Preparation 2, apart from the final product was azeotroped with diisopropyl ether.
¹H NMR (400 MHz, D₆-DMSO) □□0.72-0.86 (m, 2H), 1.03-1.27 (m, 8H), 1.24 (d, 6H), 1.51-170 (m, 7H), 2.67-2.80 (m, 2H), 2.95-3.03 (m, 1H), 3.41-3.48 (m, 1 H), 7.48 (d, 2H), 7.90 (d, 2H).
LRMS (ES) 490 (M-H).
Anal. Calcd. For C₂₄H₃₃N₃O₆S + 0.2 DIPE: C, 59.11; H, 7.05; N, 8.21. Found C, 58.93; H, 7.15; N, 8.14.

### Preparation 11

### tert-Butyl (3R)-6-cyclohexyl-3-[3-({[(3,4-dimethoxyphenyl)sulfonyl]amino} carbonyl)-1,2,4-oxadiazol-5-yl]hexanoate

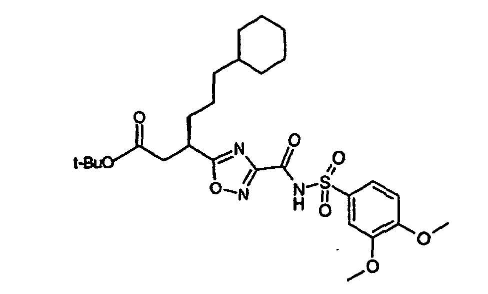

The title compound was obtained as a clear oil from the title compound from Preparation 28 and 3,4-dimethoxylbenzenesulfonyl chloride, using a similar method to that described in Preparation 1.
¹H NMR (400 MHz, D₆-DMSO) □□0.71-0.84 (m, 2H), 1.03-1.28 (m, 8H), 1.30 (s, 9H), 1.52-1.67 (m, 7H), 2.63-2.77 (m, 2H), 3.30-3.39 (m, 1H), 3.76 (s, 3H), 3.79 (s, 3H), 7.02 (d, 1H), 7.40-7.47 (m, 2H).
LRMS (ES) 564 (M-H).

### Preparation 12

### (3R)-6-Cyclohexyl-3-[3-({[(3,4-dimethoxyphenyl)sulfonyl]amino}carbonyl)-1,2,4-oxadiazol-5-yl]hexanoic acid

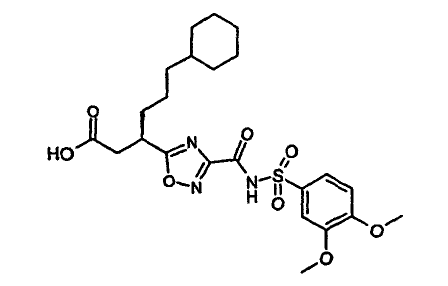

The title compound was obtained as a white foam from the title compound from Preparation 11, using a similar method to that described in Preparation 2.
¹H NMR (400 MHz, D₆-DMSO) □□0.73-0.88 (m, 2H), 1.08-1.24 (m, 8H), 1.55-1.70 (m, 7H), 2.66-2.80 (m, 2H), 3.37-3.46 (m, 1H), 3.79 (s, 3H), 3.82 (s, 3H), 7.08 (d, 1H), 7.44 (s, 1H), 7.52 (d, 1H).
LRMS (ES) 508 (M-H).

### Preparation 13

### tert-Butyl (3R)-6-cyclohexyl-3-(3-{[(8-quinolinylsulfonyl)amino]carbonyl}-1,2,4-oxadiazol-5-yl)hexanoate

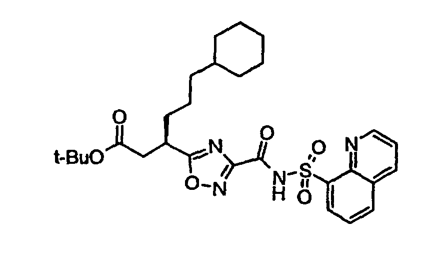

The title compound was obtained as a white solid from the title compound from Preparation 28 and 8-quinolinesulfonyl chloride, using a similar method to that described in Preparation 1.
Mpt 107-109 °C.
¹H NMR (400 MHz, D₆-DMSO) □□0.70-0.84 (m, 2H), 1.02-1.30 (m, 17H), 1.50-1.67 (m, 7H), 2.63-2.70 (m, 2H), 3.31-3.42 (m, 1H), 7.76 (dd, 1 H), 7.84 (dd, 1H), 8.38 (d, 1H), 8.51 (d, 1H), 8.66 (d, 1H), 9.07 (d, 1H).
LRMS (ES) 555 (M-H).
Anal. Calcd. For C₂₈H₃₆N₄O₆S: C, 60.41; H, 6.52; N, 10.06. Found C, 60.01; H, 6.52; N, 9.98.

### Preparation 14

### (3R)-6-Cyclohexyl-3-(3-{[(8-quinolinylsulfonyl)amino]carbonyl}-1,2,4-oxadiazol-5-yl)hexanoic acid

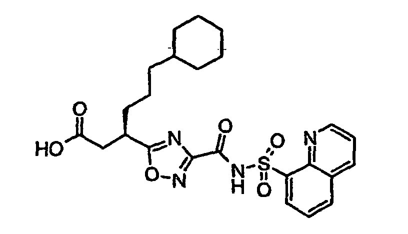

The title compound was obtained as a white solid from the title compound from Preparation 13 using a similar method to that described in Preparation 2.
Mpt 68-72 °C.
¹H NMR (400 MHz, D₆-DMSO) □□0.70-0.84 (m, 2H), 1.00-1.22 (m, 8H), 1.50-1.67 (m, 7H), 2.65-2.79 (m, 2H), 3.37-3.45 (m, 1H), 7.78 (dd, 1H), 7.87 (dd, 1H), 8.40 (d, 1H), 8.52 (d, 1H), 8.68 (d, 1H), 9.10 (d, 1H).
LRMS (ES) 499 (M-H).

### Preparation 15

### tert-Butyl (3R)-6-cyclohexyl-3-[3-({[(3,5-dimethyl-4-isoxazolyl)sulfonyl]amino} carbonyl)-1,2,4-oxadiazol-5-yl]hexanoate

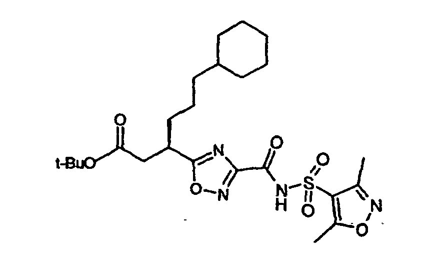

The title compound was obtained as a clear oil from the title compound from Preparation 28 and 3,5-dimethyl-4-isoxazolesulfonyl chloride, using a similar method to that described in Preparation 1.
¹H NMR (400 MHz, D₆-DMSO) □□0.74-0.85 (m, 2H), 1.03-1.30 (m, 17H), 1.53-1.67 (m, 7H), 2.30 (s, 3H), 2.58 (s, 3H), 2.66-2.74 (m, 2H), 3.34-3.45 (m, 1H).
LRMS (ES) 523 (M-H).
Anal. Calcd. For C₂₄H₃₆N₄O₇S: C, 54.95; H, 6.92; N, 10.68. Found C, 55.30 H, 7.05; N, 10.55.

### Preparation 16

### (3R)-6-Cyclohexyl-3-[3-({[(3,5-dimethyl-4-isoxazolyl)sulfonyl]amino}carbonyl)-1,2,4-oxadiazol-5-yl]hexanoic acid

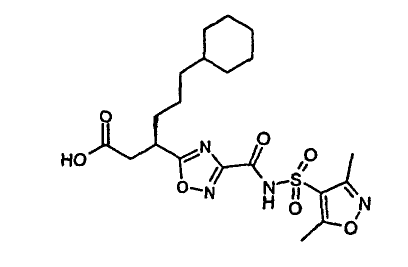

The title compound was obtained as a yellow foam from the title compound from Preparation 15, using a similar method to that described in Preparation 2, apart from the final product was azeotroped with diisopropyl ether..
¹H NMR (400 MHz, D₆-DMSO) □□0.74-0.85 (m, 2H), 1.03-1.24 (m, 8H), 1.51-1.68 (m, 7H), 2.32 (s, 3H), 2.58 (s, 3H), 2.67-2.79 (m, 2H), 3.38-3.45 (m, 1H).
Anal. Calcd. For C₂₀H₂₈N₄O₇S + 0.12 DIPE: C, 51.76; H, 6.22; N, 11.65. Found C, 51.66; H, 6.44; N, 11.27.

### Preparation 17

### tert-Butyl (3R)-6-cyclohexyl-3-(3-{[(isopropylsulfonyl)amino]carbonyl}-1,2,4-oxadiazol-5-yl)hexanoate

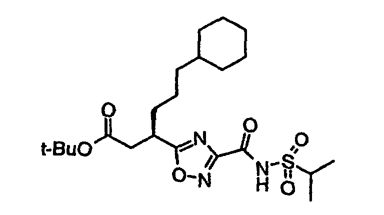

The title compound was obtained as a clear glass from the title compound from Preparation 28 and 2-propanesulfonyl chloride, using a similar method to that described in Preparation 1.
¹H NMR (400 MHz, CDCl₃) □□0.75-0.92 (m, 2H), 1.06-1.90 (m, 30H), 2.62-2.88 (m, 2H), 3.08-3.24 (m, 1H), 3.57-3.68 (m, 1H).
LRMS (ES) 470 (M-H).
Anal. Calcd. For C₂₂H₃₇N₃O₆S + 1.29 H₂O: C, 53.40; H, 8.06; N, 8.49. Found C, 53.46; H, 7.41; N, 8.41.

### Preparation 18

### (3R)-6-Cyclohexyl-3-(3-{[(isopropylsulfonyl)amino]carbonyl}-1,2,4-oxadiazol-5-yl)hexanoic acid

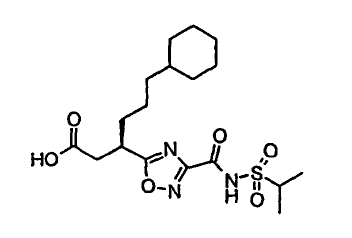

The title compound was obtained as a yellow foam from the title compound from Preparation 17, using a similar method to that described in Preparation 2, apart from the reaction was carried out in neat trifluoroacetic acid.
¹H NMR (400 MHz, CDCl₃) □□0.77-0.98 (m, 2H), 1.04-1.95 (m, 21H), 2.73-3.04 (m, 2H), 3.50-3.68 (m, 1 H), 3.78-3.95 (m, 1H).
LRMS (ES) 414 (M-H).
Anal. Calcd. For C₁₈H₂₉N₃O₆S + 0.9 TFA: C, 45.90; H, 5.82; N, 8.11. Found C, 45.96; H, 6.06; N, 8.34.

### Preparation 19

### tert-Butyl (3R)-6-cyclohexyl-3-(3-{[methyl(methylsulfonyl)amino]carbonyl}-1,2,4-oxadiazol-5-yl)hexanoate

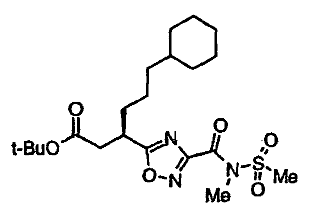

To a solution of the title compound from Preparation 29 (0.50 g, 1.3 mmol) in tetrahydrofuran (10 mL) at -78°C was added lithium bis(trimethylsilyl)amide (1.4 mL of a 1.0 mol L⁻¹ solution in tetrahydrofuran, 1.4 mmol). The reaction was stirred for 45 minutes then methanesulfonyl chloride (0.11 mL, 1.4 mmol) was added. The reaction was warmed to room temperature after 5 hours and then stirred overnight. The mixture was quenched with water (10 mL) and then extracted with ethyl acetate (2 x 20 mL). The combined organic extracts were dried (Na₂SO₄) and the solvents removed in vacuo. The residue was purified by flash chromatography on silica gel (elution with pentane/ethyl acetate 80:20) to give the title compound as a clear oil (0.42 g).
¹H NMR (400 MHz, CDCl₃) □□0.77-0.93 (m, 2H), 1.07-1.48 (m, 17H), 1.58-1.82 (m, 7H), 2.63 (dd, 1 H), 2.81 (dd, 1H), 3.32-3.59 (m, 7H).
LRMS (ES) 480 (M+Na).

### Preparation 20

### (3R)-6-Cyclohexyl-3-(3-{[methyl(methylsulfonyl)amino]carbonyl}-1,2,4-oxadiazol-5-yl)hexanoic acid

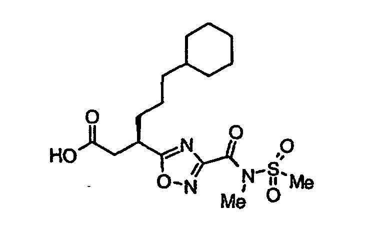

The title compound was obtained as a yellow oil from the title compound from Preparation 19, using a similar method to that described in Preparation 2, apart from the reaction was carried out in neat trifluoroacetic acid.
¹H NMR (400 MHz, CDCl₃) □□0.78-0.94 (m, 2H), 1.05-1.39 (m, 8H), 1.58-1.87 (m, 7H), 2.80 (dd, 1H), 2.98 (dd, 1 H), 3.34 (s, 3H), 3.40 (s, 3H), 3.59 (m, 1H).
LRMS (ES) 400 (M-H).
Anal. Calcd. For C₁₇H₂₇N₃O₆S: C, 50.86; H, 6.78; N, 10.47. Found C, 50.72; H, 6.83; N, 10.34.

### Preparation 21

### tert-Butyl (3R)-6-cyclohexyl-3-[3-({[(phenylmethyl)sulfonyl]amino}carbonyl)-1,2,4-oxadiazol-5-yl]hexanoate

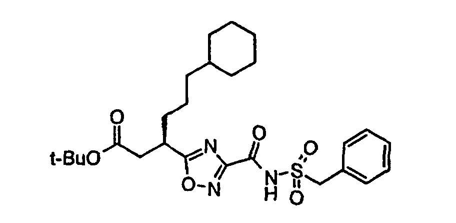

The title compound was obtained as a white foam from the title compound from Preparation 28 and phenylmethanesulfonyl chloride, using a similar method to that described in Preparation 1.
¹H NMR (400 MHz, CDCl₃) 000.78-0.92 (m, 2H), 1.07-1.81 (m, 24H), 2.52-2.84 (m, 2H), 3.47 (m, 1 H), 4.78 (s, 2H), 7.10-7.44 (m, 5H).
LRMS (ES) 518 (M-H).
Anal. Calcd. For C₂₆H₃₇N₃O₆S + 0.79 H₂O: C, 58.49; H, 7.28; N, 7.87. Found C, 58.48; H, 6.97; N, 7.78.

### Preparation 22

### (3R)-6-Cyclohexyl-3-[3-({[(phenylmethyl)sulfonyl]amino}carbonyl)-1,2,4-oxadiazol-5-yl]hexanoic acid

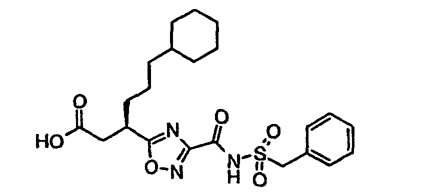

The title compound was obtained as a yellow foam from the title compound from Preparation 21, using a similar method to that described in Preparation 2, apart from the reaction was carried out in neat trifluoroacetic acid.
¹H NMR (400 MHz, CDCl₃) □□0.88-0.97 (m, 2H), 1.05-1.42 (m, 8H), 1.57-1.88 (m, 7H), 2.72-3.01 (m, 2H), 3.53 (m, 1H), 4.64-4.81 (m, 2H), 7.18-7.43 (m, 5H).
LRMS (ES) 462 (M-H).
Anal. Calcd. For C₂₂H₂₉N₃O₆S + 0.86 TFA: C, 50.73; H, 5.36; N, 7.48. Found C, 50.38; H, 5.69; N, 7.67.

### Preparation 23

### tert-Butyl (3R)-6-cyclohexyl-3-{3-[(3-pyridylsulfonyl)amino]carbonyl}-1,2,4-oxadiazol-5-yl)hexanoate

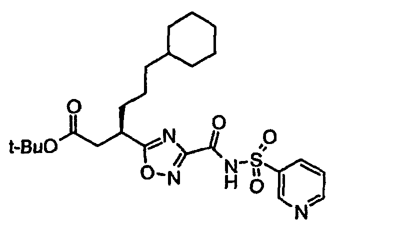

The title compound was obtained as a clear solid from the title compound from Preparation 28 and 3-pyridinesulfonyl chloride, using a similar method to that described in Preparation 3.
¹H NMR (400 MHz, CDCl₃) □□0.78-0.94 (m, 2H), 0.99-1.77 (m, 24H), 2.58-2.84 (m, 2H), 3.57 (m, 1 H), 7.29 (m, 1H), 8.25 (m, 1 H), 8.64 (m, 1 H), 9.12 (m, 1H).
LRMS (ES) 505 (M-H).
Anal. Calcd. For C₂₄H₃₄N₄O₆S + 0.43 CH₂Cl₂: C, 54.02; H, 6.47; N, 10.32. Found C, 53.99; H, 6.33; N, 10.41.

### Preparation 24

### (3R)-6-Cyclohexyl-3-{3-[(3-pyridylsulfonyl)amino]carbonyl}-1,2,4-oxadiazol-5-yl)hexanoic acid

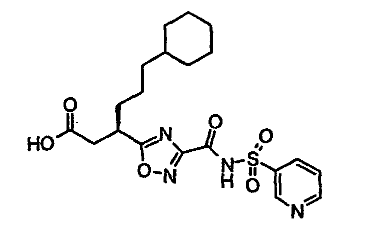

The title compound was obtained as a white solid from the title compound from Preparation 23 using a similar method to that described in Preparation 2, apart from the reaction was carried out in neat trifluoroacetic acid.
¹H NMR (400 MHz, CD₃OD) □□0.88-0.92 (m, 2H), 1.04-1.32 (m, 8H), 1.56-1.79 (m, 7H), 2.77 (dd, 1H), 2.86 (dd, 1H), 3.51 (m, 1 H), 7.62 (m, 1 H), 8.46 (d, 1H), 8.79 (d, 1 H), 9.19 (s, 1H).
LRMS (ES) 224 [(M-2H)/2].
Anal. Calcd. For C₂₀H₂₆N₄O₆S + 1.45 TFA: C, 44.66; H, 4.49; N, 9.10. Found C, 44.67; H, 4.60; N, 9.31.

### Preparation 25 : (2R)-2-[2-(tert-Butoxy)-2-oxoethyl]-5-cyclohexylpentanoic acid

### Route A:

### (2R)-2-[2-(tert-butoxy)-2-oxoethyl]-5-cyclohexylpentanoic acid

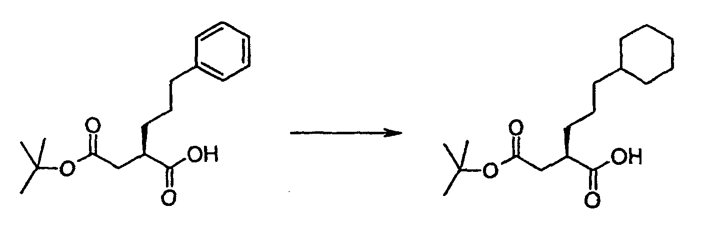

A solution of (2R)-2-[2-(tert-butoxy)-2-oxoethyl]-5-phenylpentanoic acid (Syn. Lett.; 1998; 637-639) (10.00g, 34.2mmol) in acetic acid (120ml) was treated with 5% Rhodium on alumina catalyst, pressurised to 60psi with hydrogen in a sealed vessel and stirred at room temperature for 17 hours. The mixture was filtered through a pad of Arbocel® and the solvent was removed from the filtrate under reduced pressure. The residue was azeotroped from toluene to afford the title compound (7.53g).
MS : 299 (MH⁺)
¹H-NMR (CDCl₃) δ : 2.80 (1H, m), 2.61 (1H, m), 2.38 (1H, m), 1.75-1.56 (7H, m), 1.55-1.04 (17H, m), 0.84 (2H, m).

### Route B:

### (4S)-4-Benzyl-3-(5-cyclohexylpentanoyl)-1,3-oxazolidin-2-one

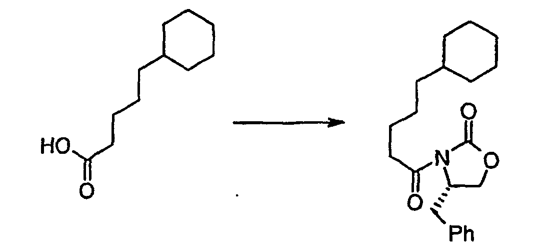

A solution of 5-cyclohexylpentanoic acid (63.50g, 345mmol) in N,N-dimethylformamide (0.5ml) and dichloromethane (350ml) was cooled to 5°C and treated dropwise with oxalyl chloride (31.6ml, 362mmol) over 30 minutes. The mixture was stirred at 0°C for 3 hours then the solvent was removed under reduced pressure to afford 5-cyclohexylpentanoyl chloride as a pale yellow solid (70.0g).
A solution of n-butyllithium (100ml, 250mmol, 2.5M in hexanes) was added via a cannula to a solution of (4S)-4-benzyl-1,3-oxazolidin-2-one (44.30g, 250mmol) in anhydrous tetrahydrofuran (400ml) at -78°C. The yellow solution was then stirred for 45 minutes. A solution of 5-cyclohexylpentanoyl chloride (55.5g, 275mmol) in tetrahydrofuran (100ml) was then added over 1 hour. The mixture was stirred at -78°C for 30 minutes then warmed to room temperature over 1 hour. The mixture was quenched with an aqueous solution of ammonium chloride (20% w/v, 400ml) and extracted with ethyl acetate. The layers were separated and the organic layer was washed with brine, dried over anhydrous sodium sulphate and the solvent removed under reduced pressure. The solid was recrystallised from hexane (500ml) to afford the title compound as a white solid (81.0g).
MS: 344 (MH⁺)
¹H-NMR (CDCl₃) δ : 7.41-7.13 (5H, m), 4.68 (1H, m), 4.27-4.02 (2H, m), 3.31 (1 H, dd, J=16, 4Hz), 3.06-2.70 (3H, m), 1.81-1.53 (7H, m), 1.49-1.04 (8H, m), 0.88 (2H, m)

### tert-Butyl 3-{[(4S)-4-benzyl-2-oxo-1,3-oxazolidin-3-yl]carbonyl}-6-cyclohexylhexanoate

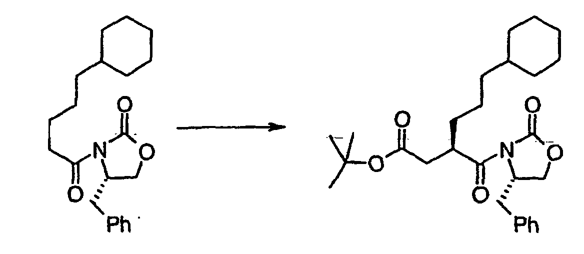

A solution of (4S)-4-benzyl-3-(5-cyclohexylpentanoyl)-1,3-oxazolidin-2-one (70.0g, 204mmol) in anhydrous tetrahydrofuran (650ml) was cooled to -70°C and treated dropwise with sodium hexamethyldisilazide (1M in tetrahydrofuran, 224ml, 224mmol) over 45 minutes. The mixture was stirred for a further 45 minutes before being treated with t-butylbromoacetate (31.6ml, 214mmol). This mixture was stirred at -70°C for 30 minutes then warmed to -30°C and quenched with an aqueous solution of ammonium chloride (20%w/v, 400ml) and warmed to room temperature. The mixture was extracted with ethyl acetate and the combined organic extracts were washed with brine, dried over anhydrous sodium sulphate and the solvent removed under reduced pressure. The solid was recrystallised from hexane to afford the title compound as a white solid (71.4g).
MS: 458(MH⁺)
¹H-NMR (CDCl₃) δ : 7.41-7.13 (5H, m), 4.66 (1H, m), 4.23-4.03 (3H, m), 3.35 (1H, dd, J=16, 4Hz), 2.95-2.68 (3H, m), 2.47 (1H, m), 1.80-1.07 (24H, m), 0.85 (2H, m)

### 2-[2-(tert-Butoxy)-2-oxoethyl]-5-cyclohexylpentanoic acid

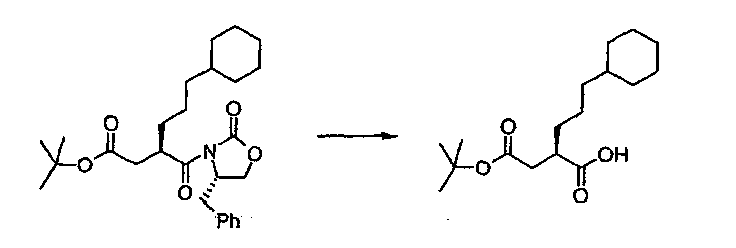

A solution of tert-butyl 3-{[(4S)-4-benzyl-2-oxo-1,3-oxazolidin-3-yl]carbonyl}-6-cyclohexylhexanoate (64.0g, 139.9mmol) in tetrahydrofuran: water (3:1, 800ml) was cooled to 5°C then treated sequentially with hydrogen peroxide (30%w/v water, 87ml, 769mmol) then lithium hydroxide hydrate (10.0g, 238mmol). The reaction was stirred for 1 hour then quenched by dropwise addition of an aqueous solution of sodium thiosulphate (500ml) keeping the temperature below 20°C. The mixture was extracted with ethyl acetate (discarded) and the aqueous phase was acidified to pH 2 with solid citric acid and extracted with ethyl acetate. The combined organic phases were washed with brine, dried over anhydrous sodium sulphate and the solvent removed under reduced pressure. The residue was purified by column chromatography on silica gel eluting with a gradient system of hexane : ethyl acetate (2 : 1) gradually changing to hexane : ethyl acetate (1 : 1) to afford the title compound (40.7g)

### Route C:

### 3-(Diethoxyphosphoryl)succinic acid 1-tert-butyl ester

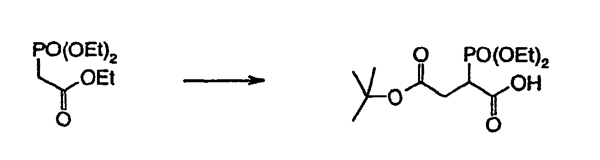

Triethylphosphonoacetate (102g, 0.45 mol) was added dropwise over 11 min to a stirred solution of potassium tert-butoxide (60g, 0.54 mol) in THF (500ml), at 0°C, under nitrogen. The mixture was stirred for 1 hour at 0°C and then dichloromethane (300ml) was added and the reaction mixture was warmed to 25-30°C. The mixture was stirred at 25-30°C for 1 hour and then added dropwise over 33 minutes to a solution of tert-butyl bromoacetate (96g, 0.49 mol) in THF (500ml), at 0°C, under nitrogen. The mixture was stirred at 0-5°C for 2 hours and then a solution of citric acid (174g, 0.91 mol) in demineralised water (250ml) was added. The mixture was concentrated in vacuo to remove most of the THF and then toluene (750ml) was added. The organic phase was separated, washed with brine (2x150ml) and concentrated in vacuo to leave a colourless oil. The oil was taken up in ethanol and a solution of potassium hydroxide (36.g, 0.64mol) in demineralised water (150ml) was added dropwise over 15 mins. The mixture was stirred at 0°C for 4 hours and then a solution of citric acid (158 g, 0.82 mol) in demineralised water (600ml), and toluene (600ml), were added. The organic phase was separated and the aqueous phase was re-extracted with toluene (600ml). The combined organic phases were washed with demineralised water (2x150ml) and concentrated in vacuo to leave a white solid. Toluene (150ml) was added and the slurry was re-concentrated in vacuo to leave a white solid. The product was purified by crystallisation from tert-butylmethyl ether (300ml) and cyclohexane (600ml) to give the title compound as a solid (79g).
¹H-NMR (CDCl₃) δ : 4.20-4.10 (4H, m), 3.49-3.36 (1H, m), 3.00-2.85 (1H, m), 2.72-2.60 (1H, m), 1.20 (9H, s), 1.37-1.27 (6H, m)

### Alternative preparation:

Triethylphosphonoacetate (12.0Kg, 53.5 mol) was added over 30 minutes to a stirred solution of potassium *tert*-butoxide (7.20Kg, 64.2 mol) in THF (118 litres), between 0 and 5°C, under nitrogen. The mixture was warmed to 25-30°C where it was stirred for 1 hour and then added over 45 minutes to a solution of *tert*-butyl bromoacetate (11.5Kg, 59.0 mol) in THF (28 litres), between 0 and 5°C, under nitrogen. The mixture was stirred at 0-5°C for 1 hour and then demineralised water (6.1 litres) and ethanol (30 litres) were added. A solution of potassium hydroxide (4.2Kg, 75.0 mol) in demineralised water (84 litres) was then added over 2 hours, between -5 and 0°C. The mixture was stirred at -10°C for 16 hours and then a solution of citric acid (16.5Kg, 85.8 mol) in demineralised water (32 litres) was added. The mixture was concentrated in vacuo to a volume of 180 litres and then ethyl acetate (90 litres) was added. The organic phase was separated and the aqueous phase was re-extracted with ethyl acetate (30 litres). The combined organic phases were washed with water (30 litres) and then stripped and replaced with cyclohexane by distillation at atmospheric pressure, at a constant volume of 72 litres. *tert*-Butylmethyl ether (18 litres) was added and the mixture was stirred at ambient temperature for 12 hours and then filtered. The residue was washed with a mixture of cyclohexane (16 litres) and *tert*-butylmethyl ether (3.6 litres) then dried *in vacuo* for 16 hours to give the title compound as a colourless solid (10.0Kg, 60%).
¹H-NMR (CDCl₃) δ : 4.20-4.10 (4H, m), 3.49-3.36 (1H, m), 3.00-2.85 (1 H, m), 2.72-2.60 (1H, m), 1.20 (9H, s), 1.37-1.27 (6H, m)

### (E)-2-[2-(tert-Butoxy)-2-oxoethyl]-5-phenyl-2-pentenoic acid

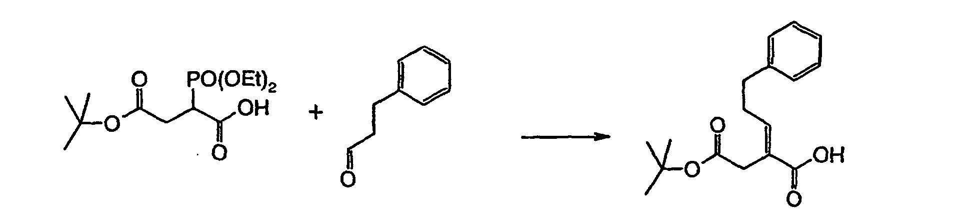

A solution of 3-(diethoxyphosphoryl)succinic acid 1-*tert*-butyl ester (100g, 0.32mol) in THF (300ml) was added dropwise over 15 min to a stirred solution of potassium *tert*-butoxide (110g, 0.98mol) in THE (300ml), between -10 and -5°C, under nitrogen. The mixture was stirred at -10°C for 15 min and then a solution of hydrocinnamaidehyde (46.8g, 0.35mmol) in THF (100ml) was added dropwise over 15 min, between -13 and -8°C. The mixture was stirred at -10°C for 30 min and then a solution of citric acid (111g, 0.58mol) in demineralised water (500ml), and ethyl acetate (500ml), were added. The pH was adjusted to pH 4 with aqueous sodium hydroxide solution (50%) and the phases were separated. The aqueous fraction was washed with ethyl acetate (500ml) and the combined organic fractions were washed with saturated sodium bicarbonate solution (500ml), citric acid solution (10%, 500ml) and demineralised water (500ml) and then concentrated *in vacuo.* The resulting solid was slurried in cyclohexane (470ml) for 1 hour and then the mixture was filtered. The residue was washed with cyclohexane (2x50ml) and dried *in vacuo* to leave the title compound as a colourless solid (76g, 81%).
MS: 289 [(M-H)]⁻
¹H-NMR (CDCl₃) δ : 7.33-7.16 (5H, m), 7.05 (1H, br t), 3.20 (2H, s), 2.89 (2H, br t), 2.50 (2H, br dd), 1.41 (9H, s)

### (R)-2-[2-(tert-Butoxy)-2-oxoethyl]-5-phenylpentanoic acid

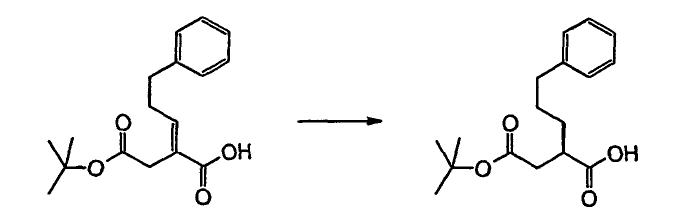

A stirred solution of (E)-2-[2-(tert-butoxy)-2-oxoethyl]-5-phenyl-2-pentenoic acid (100g, 0.34mol), cyclohexylamine (39ml, 0.34mol) and [(S)-2,2'-bis(diphenylphosphino-1,1'-binaphthyl]chloro(p-cymene)ruthenium chloride (0.64g, 0.69mmol) in methanol (1000ml) was heated to 60°C, under hydrogen (60p.s.i.), for 42 hours and then allowed to cool to room temperature. The mixture was filtered through celite and then concentrated in vacuo to a yellow solid which was purified by re-crystallisation from acetone (850ml). The resulting solid was partitioned between ethyl acetate (1200ml) and citric acid solution (10%, 1200ml) and the organic phase was separated, washed with demineralised water (1200ml) and concentrated in vacuo to leave the title compound as an oil (80g).
¹H-NMR (CDCl₃) δ : 7.30-7.17 (5H, m), 2.85-2.78 (1H, m), 2.66-2.58 (3H, m), 2.37 (1H, br dd), 1.75-1.51 (4H, m), 1.40 (9H, s)

### Preparation of cyclohexylamine salt:

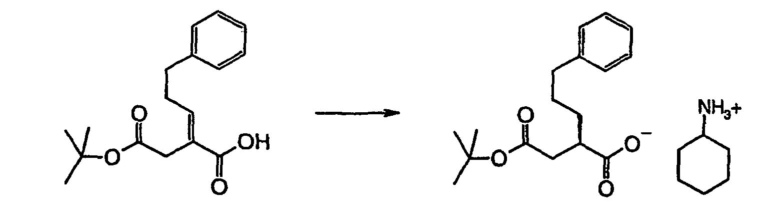

A stirred solution of cyclohexylamine (266ml, 2.32 mol), (*E*)-2-[2-(*tert*-butoxy)-2-oxoethyl]-5-phenyl-2-pentenoic acid (688g, 2.37 mol) and [(*S*)-2,2'-bis(diphenylphosphino-1,1'-binaphthyl]chloro(*p*-cymene)ruthenium chloride (4.4g, 4.7 mmol) in methanol (6.9 litres) was heated to 60°C, under hydrogen (60p.s.i.), for 47 hours and then allowed to cool to room temperature (enantiomeric excess= 88%). The mixture was filtered through celite and then the solvent was stripped and replaced with acetone by distillation at atmospheric pressure, at a constant volume of 4.2 litres. The resulting suspension was cooled to room temperature where it was stirred for 4 hours and then filtered. The residue was washed with acetone (2x 1 litre) and then dried *in vacuo* at 45°C for 16 hours to leave the title compound as a colourless solid (590g, 64%, enantiomeric excess=98.9%).
¹H-NMR (CD₃OD) δ : 7.23-7.09 (5H, m), 3.05-2.98 (1H, m), 2.64-2.56 (3H, m), 2.53 (1H, dd, J 15.2, 7.2Hz), 2.23 (1H, dd, J 15.2, 7.2Hz), 2.00-1.97, (2H, m), 1.85-1.81 (2H, m), 1.72-1.20 (10H, m), 1.40 (9H, s)

### (R)-2-[2-(tert-Butoxy)-2-oxoethyl]-5-cyclohexylpentanoic acid cyclohexylamine salt

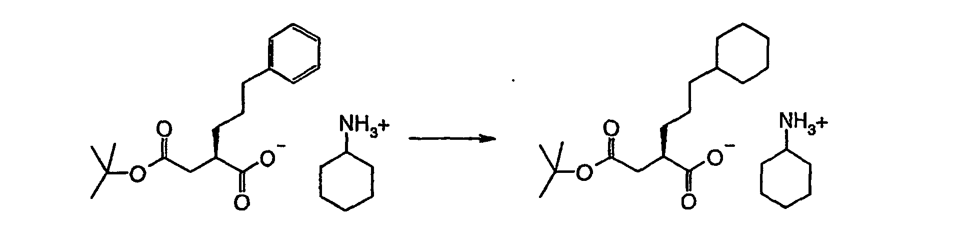

(*R*)-2-[2-(*tert*-Butoxy)-2-oxoethyl]-5-phenylpentanoic acid cyclohexylamine salt (691g, 1.77 mol) and ethyl acetate (7.0 litres) were added to an aqueous solution of citric acid (10%, 6.3 litres) and the organic phase was separated, washed with water (7.0 litres) and concentrated *in vacuo* to a yellow oil. A solution of the oil and 5% rhodium on carbon (51.6g) in methanol (7.0 litres) was stirred at ambient temperature, under hydrogen (150p.s.i.) for 48 hours and then filtered through celite. To the filtrate was added cyclohexylamine (202ml, 1.77 mol) and the methanol solution was stripped and replaced with methylethyl ketone by distillation at atmospheric pressure, to a volume of 5.5 litres. The mixture was allowed to cool to ambient temperature where it was stirred for 48 hours and then filtered. The residue was washed with methylethyl ketone (2x 500ml) and then dried *in vacuo* at 45°C for 4 hours to leave the title compound as a colourless solid (495g, 71 %).
¹H-NMR (CD₃OD) δ : 3.06-2.99 (1H, m), 2.63-2.56 (1 H, m), 2.53 (1H, dd, J 15.2, 7.2Hz), 2.23 (1H, dd, J 15.2, 7.2Hz), 2.02-1.97 (2H, m), 1.77-1.15 (21H, m), 1.43 (9H, s), 0.93-0.82 (2H, m)

### (R)-2-[2-(tert-Butoxy)-2-oxoethyl]-5-cyclohexylpentanoic acid

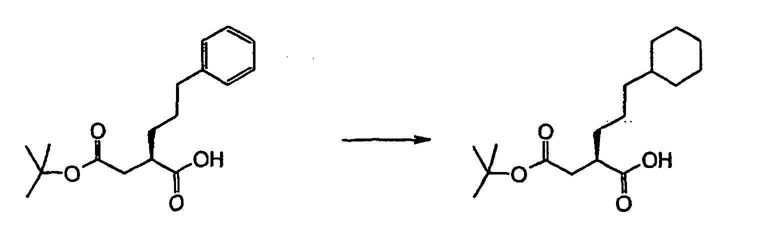

A solution of (R)-2-[2-(tert-butoxy)-2-oxoethyl]-5-phenylpentanoic acid (2.2g, 7.5mmol) and 5%Rh/C (0.22g) in methanol (220ml) was stirred at room temperature, under hydrogen (150p.s.i.) for 24 hours and then filtered through celite. The filtrate was concentrated in vacuo to leave the title compound as an oil (2.0g).
¹H-NMR (CDCl₃) δ : 2.82-2.76 (1H, m), 2.60 (1H, br dd), 2.37 (1H, br dd), 1.70-1.60 (6H, m), 1.51-1.30 (3H, m), 1.42 (9H, s), 1.23-1.11 (6H, m), 0.96-0.80 (2H, m)

### Preparation 26:

### tert-Butyl (3R)-3-[({[(Z)-1-amino-2-ethoxy-2-oxoethylidene]amino}oxy)carbonyl]-6-cyclohexylhexanoate

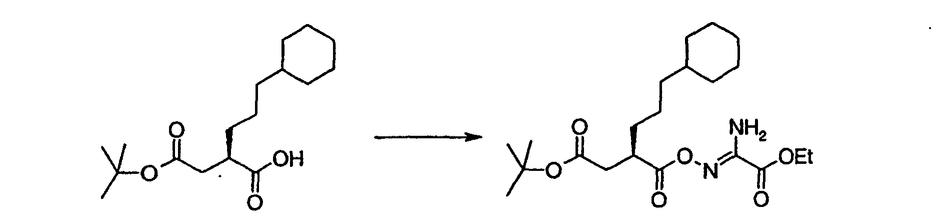

A solution of (2R)-2-[2-(tert-butoxy)-2-oxoethyl]-5-cyclohexylpentanoic acid (Preparation 25) (7.53g, 25.2mmol) in 1,4-dioxane (175ml) was treated with 1-hydroxybenzotriazole hydrate (3.75g, 27.8mmol) and the mixture cooled to 0°C. N,N'-Dicyclohexylcarbodiimide (5.47g, 26.5mmol) was then added and the mixture was stirred for 3 hours being allowed to warm to room temperature over this time. The mixture was then filtered and washed with 1,4-dioxane (2x50ml). The filtrate was then treated with sodium carbonate (4.01g, 37.8mmol) and ethyl 2-amino-2-(hydroxyimino)acetate (J.Org.Chem.;23; 1958; 1794) (3.33g, 25.2mmol). The resulting mixture was stirred at room temperature for 17 hours. The solvent was removed under reduced pressure and the residue was partitioned between ethyl acetate and water. The layers were separated and the aqueous phase was extracted with ethyl acetate (x2). The combined organic layers were dried over anhydrous magnesium sulphate, filtered and the solvent removed under reduced pressure. The residue was purified by column chromatography on silica gel eluting with a gradient system of ethyl acetate : pentane (30 : 70) gradually changing to ethyl acetate : pentane (50 : 50) to afford the title compound as a white solid (6.50g).
MS : 413 (MH⁺)
¹H-NMR (CDCl₃) δ : 5.71 (2H, br s), 4.39 (2H, q), 2.92 (1H, m), 2.67 (1H, dd), 2.44 (1H, dd), 1.75-1.32 (22H, m), 1.26-1.04 (5H, m), 0.84 (2H, m).

### Preparation 27

### Ethyl 5-{(1R)-1-[2-(tert-butoxy)-2-oxoethyl]-4-cyclohexylbutyl}-1,2,4-oxadiazole-3-carboxylate

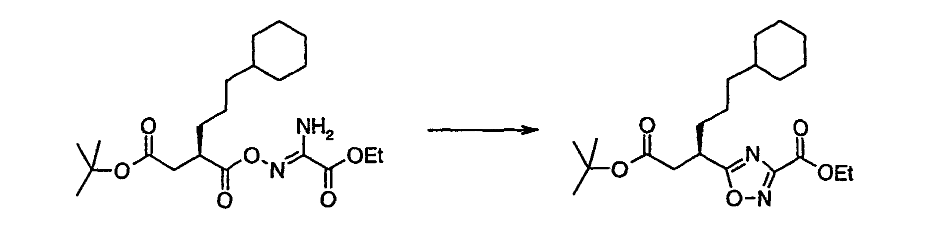

A solution of tert-Butyl (3R)-3-[({[(Z)-1-amino-2-ethoxy-2-oxoethylidene]amino}oxy)carbonyl]-6-cyclohexylhexanoate (Preparation 26) (21.0g, 50.82mmol) in xylene (400ml) was heated at 130°C for 17 hours, then allowed to cool to room temperature. The residue was purified by column chromatography on silica gel eluting with a gradient system of ethyl acetate: pentane (5 : 95) gradually changing to ethyl acetate : pentane (20 : 80) to afford the title compound as a colourless oil (20.0g).
MS : 395 (MH⁺), 412 (MNH₄⁺)
¹H-NMR (CDCl₃) 8: 4.51 (2H, m), 3.54 (1H, m), 2.86 (1H, dd), 2.65 (1H, dd), 1.86-1.57 (7H, m), 1.50-1.33 (12H, m), 1.30-1.03 (8H, m), 0.82 (2H, m).

### Preparation 28

### tert-Butyl (3R)-3-[3-(aminocarbonyl)-1,2,4-oxadiazol-5-yl]-6-phenylhexanoate

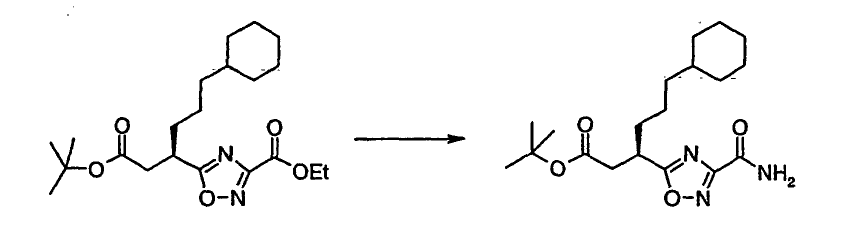

A solution of ethyl 5-{(1R)-1-[2-(tert-butoxy)-2-oxoethyl]-4-cyclohexylbutyl)-1,2,4-oxadiazole-3-carboxylate (Preparation 27) (400mg, 1.01mmol) in ethanol saturated with ammonia gas (20ml) was stirred at room temperature for 18 hours. The solvent was removed under reduced pressure and the residue was purified by column chromatography on silica gel eluting with a gradient system of hexane : ethyl acetate (90 : 10) gradually changing to hexane : ethyl acetate (60 : 40) to afford the title compound as a white solid (260mg).
MPt : 77-79°C
MS: 366 (MH⁺), 383 (MNa⁺)
Analysis: Found C, 62.42; H, 8.59; N, 11.48%; C₁₉H₃₁N₃O₄ requires C, 62.44; H, 8.55; N, 11.50%
¹H-NMR (CDCl₃) δ : 6.80 (1H, br s), 5.90 (1H, br s), 3.53 (1 H, m), 2.87 (1H, dd, J=17, 9Hz), 2.66 (1H, dd, J=17, 5Hz), 1.90-1.50 (7H, m), 1.46-1.02 (17H, m), 0.83 (2H, m).

### Preparation 29

### tert-Butyl (3R)-6-cyclohexyl-3-{3-[(methylamino)carbonyl]-1,2,4-oxadiazol-5-yl}hexanoate

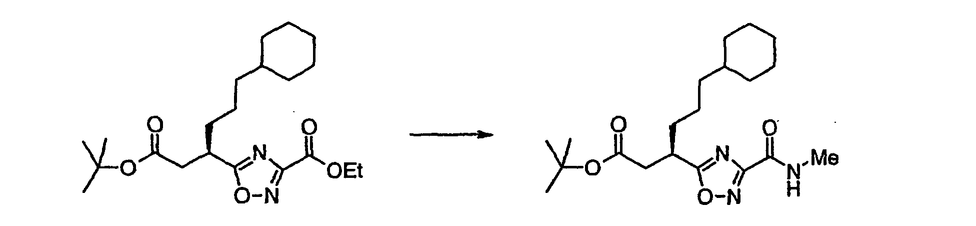

A solution of ethyl 5-{(1R)-1-[2-(tert-butoxy)-2-oxoethyl]-4-cyclohexylbutyl}-1,2,4-oxadiazole-3-carboxylate (Preparation 27) (4.70g, 11.9mmol) in ethanol (80ml) was treated with methylamine (33% w/v in ethanol, 12.0ml, 96.0mmol) and the solution was stirred at room temperature for 18 hours. The solvent was removed under reduced pressure and the residue was purified by column chromatography on silica gel eluting with a gradient system of dichloromethane : ethyl acetate (9 : 1) gradually changing to dichloromethane : ethyl acetate (8 : 2) to afford the title compound as a pale yellow oil which crystallised on standing (4.23g).
MS : 380 (MH⁺)
¹H-NMR (CDCl₃) δ : 6.97 (1H, br m), 3.48 (1H, m), 3.04 (3H, d), 2.84 (1H, dd, J=17, 9Hz), 2.66 (1 H, dd, J=17, 4Hz), 1.84-1.55 (7H, m), 1.39 (9H, s), 1.33-1.02 (8H, m), 0.83 (2H, m).

### Preparation 30

### tert-Butyl (3R)-6-cyclohexyl-3-({[(1S)-2-ethoxy-1-(hydroxymethyl)-2-oxoethyl]amino}carbonyl)hexanoate

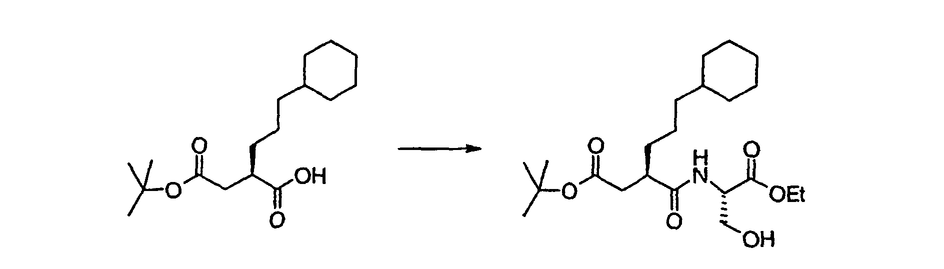

A solution of (2R)-2-[2-(tert-butoxy)-2-oxoethyl]-5-cyclohexylpentanoic acid (Preparation 25) (5.00g, 16.76mmol) in dichloromethane (75ml) was treated sequentially with 1-hydroxybenzotriazole hydrate (2.49g, 18.43mmol), serine ethyl ester hydrochloride (3.13g, 18.43mmol) and N,N-diisopropylethylamine (6.13ml, 35.19mmol) and the resulting mixture was stirred at 0°C under a nitrogen atmosphere for 15 minutes. 1-[3-(dimethylamino)propyl]-3-ethylcarbodiimide hydrochloride (3.53g, 18.43mmol) was then added and the mixture was stirred for 48 hours being allowed to warm to room temperature over this time. The mixture was diluted with dichloromethane (200ml), washed sequentially with water, aqueous citric acid solution (10% w/v), a saturated aqueous solution of sodium hydrogen carbonate and brine, dried over anhydrous magnesium sulphate, filtered and the solvent removed under reduced pressure. The residue was then purified by column chromatography on silica gel eluting a gradient system of ethyl acetate: pentane (10 : 90) to (50 : 50) to afford the title compound as a colourless oil (5.41 g).
MS : 413 (M⁺)
Analysis: Found C, 63.20; H, 9.52; N, 3.27%; C₂₂H₃₉NO₆. 0.33 EtOAc requires C, 63.28; H, 9.48; N, 3.16%
¹H-NMR (CDCl₃) δ : 6.50 (1H, br d, J=6Hz), 4.60 (1H, m), 4.26 (2H, q, J=8Hz), 4.09 (1H, m), 3.85 (1H, m), 3.18 (1H, m), 2.70 (1H, dd, J=18, 9Hz), 2.51 (1H, m), 2.37 (1H, dd, J=18, 3Hz), 1.78-1.52 (7H, m), 1.50-1.02 (20H, m), 0.85 (2H, m)

### Preparation 31

### Ethyl (4S)-2-{(1R)-1-[2-(tert-butoxy)-2-oxoethyl]-4-cyclohexylbutyl}-4,5-dihydro-1,3-oxazole-4-carboxylate

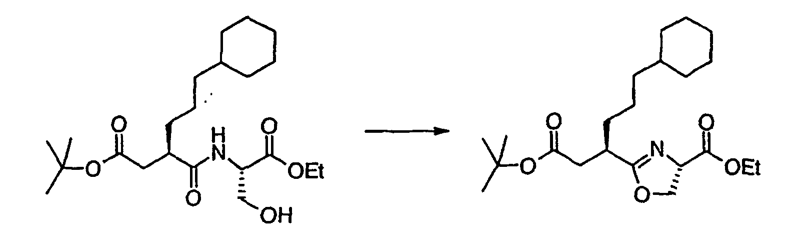

A solution of tert-butyl (3R)-6-cyclohexyl-3-({[(1S)-2-ethoxy-1-(hydroxymethyl)-2-oxoethyl]amino}carbonyl)hexanoate (Preparation 30) (4.14g, 10mmol) in anhydrous tetrahydrofuran (40ml) was treated with (methoxycarbonylsulfamoyl)triethylammonium hydroxide, inner salt [Burgess Reagent] (2.62g, 11mmol) and the resulting mixture was heated under reflux under a nitrogen atmosphere for 1 hour. The solvent was removed under reduced pressure and the residue purified by column chromatography on silica gel eluting with a gradient system of pentane : ethyl acetate (80 : 20) to (50 : 50) to afford the title compound as a colourless oil (3.10g)
¹H-NMR (CDCl₃) δ : 4.69 (1H, m), 4.52-4.33 (2H, m), 4.22 (2H, m), 2.87 (1H, m), 2.63 (1H, dd, J=16, 7Hz), 2.40 (1H, dd, J=16, 6Hz), 1.76-1.03 (27H, m), 0.85 (2H, m)

### Preparation 32

### Ethyl 2-{(1R)-1-[2-(tert-butoxy)-2-oxoethyl]-4-cyclohexylbutyl}-1,3-oxazole-4-carboxylate

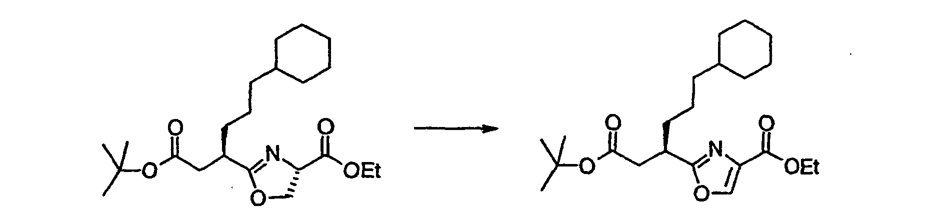

A suspension of copper (II) bromide (2.08g, 9.31mmol) and hexamethylenetetramine (1.30g, 9.31 mmol) in degassed dichloromethane (25ml) was treated with 1,8-diazabicyclo[5.4.0)undec-7-ene (1.39ml, 9.31mmol) and then cooled in a cold water bath and stirred for 5 minutes. This suspension was then treated dropwise with a solution of ethyl (4S)-2-{(1R)-1-[2-(tert-butoxy)-2-oxoethyl]-4-cyclohexylbutyl]-4,5-dihydro-1,3-oxazole-4-carboxylate (Preparation 31) (0.92g, 2.33mmol) in dichloromethane (5ml) and the resulting mixture was stirred at room temperature under a nitrogen atmosphere for 17 hours. The solvent was removed under reduced pressure and the residue partitioned between ethyl acetate and a solution of 0.88 ammonia : saturated aqueous solution of ammonium chloride (1 : 1, 100mls). The layers were separated and the aqueous layer was extracted with ethyl acetate (x2). The organic layers were combined, washed sequentially with hydrochloric acid (2M), saturated aqueous sodium hydrogen carbonate solution and brine, dried over anhydrous magnesium sulphate, filtered and the solvent removed under reduced pressure. The residue was purified by column chromatography on silica gel eluting with ethyl acetate: pentane (10 : 90) to afford the title compound as a pale yellow oil (0.59g).
MS : 394 (MH⁺)
¹H-NMR (CDCl₃) δ : 8.13 (1H, s), 4.39 (2H, q, J=7Hz), 3.39 (1H, m), 2.80 (1H, dd, J=17, 8Hz), 2.58 (1H, dd, J=17, 6Hz),1.84-1.53 (7H, m), 1.49-1.02 (20H, m), 0.84 (2H, m)

## Claims

1. A compound of formula I: 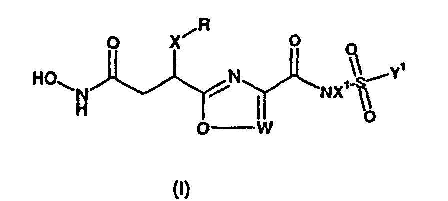 wherein:
X is C₁₋₆ alkylene or C₂₋₆ alkenylene, each of which is optionally substituted by one or more fluorine atoms;
R is aryl, C₃₋₈ cycloalkyl or C₅₋₆cycloalkenyl optionally substituted by one or more fluorine atoms.
W is N or CZ;
Z is H, or C₁-C₄ alkyl optionally substituted with halogen.
X¹ is H or C₁-C₄ alkyl,
Y¹ is:
C₁-C₄ alkyl, optionally substituted by aryl, or by one or more halogen atoms, with the proviso that when Y¹ is methyl, X¹ is not H,
or Y¹ is aryl, or a mono or bicyclic non-aromatic carbocyclic or heterocyclic moiety containing up to 10 ring atoms and which can include up to 3 ring heteroatoms, independently selected from N, O and S, which ring moiety is optionally substituted by one or more substituents independently selected from halogen, C₁-C₄ alkoxy and C₁-C₄ alkyl optionally substituted by one or more halogen.
"Aryl" is a mono or bicyclic aromatic carbocyclic or heterocyclic moiety containing up to 10 ring atoms, and which can include up to 3 ring heteroatoms, independently selected from N, O and S, which ring moiety is optionally substituted by one or more substituents, independently selected from halogen, C₁-C₄ alkoxy and C₁-C₄ alkyl optionally substituted by halogen.
and the pharmaceutically acceptable salts or solvates (including hydrates) thereof.

2. A compound according to claim 1 wherein the stereochemistry is as defined in formula (IA):

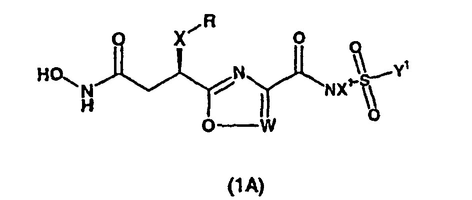

3. A compound, salt or solvate according to claim 1 or claim 2 wherein X is a linear C₂₋₄ alkylene moiety optionally substituted by one or more fluorine atoms.

4. A compound, salt or solvate according to claim 3 wherein X is propylene.

5. A compound, salt or solvate according to any preceding claim wherein R is C₃₋₈ cycloalkyl optionally substituted by one or more fluorine atoms.

6. A compound, salt or solvate according to claim 5 wherein R is cyclobutyl or cyclohexyl optionally substituted by one or more fluorine atoms.

7. A compound, salt or solvate according to claim 6 wherein R is cyclobutyl or cyclohexyl.

8. A compound, salt, or solvate according to claim 7 wherein R is cyclohexyl.

9. A compound, salt, or solvate according to any preceding claim wherein W is N, CH or CCH₃.

10. A compound, salt, or solvate according to claim 9 wherein W is N.

11. A compound, salt, or solvate according to any preceding claim wherein Y¹ is C₁-C₄ alkyl (optionally substituted by phenyl or by one or more halogen atoms), phenyl (optionally substituted by one or more substituents independently selected from halogen, C₁-C₄ alkoxy, C₁-C₄ alkyl optionally substituted with one or more halogen, and which phenyl ring is optionally pyrido-fused), a 5-or 6- membered heterocyclic ring (with one or two ring heteroatoms selected from N, O and S, which heterocyclic ring is optionally substituted by one or more substituents independently selected from halogen, C₁-C₄ alkoxy, C₁-C₄ alkyl optionally substituted with one or more halogen).

12. A compound, salt or solvate according to claim 11 wherein Y¹ is phenyl, 4-methylphenyl, 4-methoxyphenyl, 4-fluorophenyl, 4-isopropylphenyl, 3,4-dimethoxyphenyl, 8-quinolinyl, 3,5-dimethyl-4-isoxazolyl, isopropyl, methyl, benzyl or 3-pyridyl.

13. A compound, salt, or solvate according to claim 12 wherein Y¹ is phenyl, benzyl, 3,4-dimethoxyphenyl, or 3-pyridyl.

14. A compound, salt, or solvate according to claim 13 wherein Y¹ is phenyl.

15. A compound, salt, or solvate according to any preceding claim wherein X¹ is H or methyl.

16. A compound, salt, or solvate according to claim 15 wherein X¹ is H.

17. A compound, salt, or solvate of formula (I) according to claim 1 wherein the substituents are as specified in the compounds of the Examples described herein and the salts or solvates thereof.

18. A compound according to claim 1 which is selected from:
(3R)-6-Cyclohexyl-N-hydroxy-3-(3-{[(phenylsulfonyl)amino]carbonyl}-1,2,4-oxadiazol-5-yl)hexanamide;
(3R)-6-Cyclohexyl-N-hydroxy-3-[3-({[(4-methylphenyl)sulfonyl]amino}carbonyl)-1,2,4-oxadiazol-5-yl]hexanamide;
(3R)-6-Cyclohexyl-N-hydroxy-3-[3-({[(4-methoxyphenyl)sulfonyl]amino}carbonyl)-1,2,4-oxadiazol-5-yl]hexanamide;
(3R)-6-Cyclohexyl-3-[3-({[(4-fluorophenyl)sulfonyl]amino}carbonyl)-1,2,4-oxadiazol-5-yl]-N-hydroxyhexanamide;
(3R)-6-Cyclohexyl-N-hydroxy-3-[3-({[(4-isopropylphenyl)sulfonyl]amino}carbonyl)-1,2,4-oxadiazol-5-yl]hexanamide;
(3R)-6-Cyclohexyl-3-[3-({[(3,4-dimethoxyphenyl)sulfonyl]amino}carbonyl)-1,2,4-oxadiazol-5-yl]-N-hydroxyhexanamide;
(3R)-6-Cyclohexyl-N-hydroxy-3-(3-{[(8-quinolinylsulfonyl)amino]carbonyl}-1,2,4-oxadiazol-5-yl)hexanamide;
(3R)-6-Cyclohexyl-3-[3-({[(3,5-dimethyl-4-isoxazolyl)sulfonyl]amino}carbonyl)-1,2,4-oxadiazol-5-yl]-N-hydroxyhexanamide;
(3R)-6-Cyclohexyl-N-hydroxy-3-(3-{[(isopropylsulfonyl)amino]carbonyl}-1,2,4-oxadiazol-5-yl)hexanamide;
(3R)-6-Cyclohexyl-N-hydroxy-3-(3-{[methyl(methylsulfonyl)amino]carbonyl}-1,2,4-oxadiazol-5-yl]hexanamide;
(3R)-6-Cyclohexyl-N-hydroxy-3-(3-{[(phenylmethyl)sulfonyl]amino}carbonyl)-1,2,4-oxadiazol-5-yl)hexanamide;
(3R)-6-Cyclohexyl-N-hydroxy-3-(3-{[(3-pyridylsulfonyl)amino]carbonyl}-1,2,4-oxadiazol-5-yl)hexanamide;
and the salts, or solvates thereof.

19. A compound according to any one of claims 1 to 18 including the salts, or solvates thereof, for use in medicine.

20. A compound according to any one of claims 1 to 18 including the salts, or solvates thereof, for use in the treatment of a PCP mediated condition or disease.

21. The use of a compound according to any one of claims 1 to 18 including the salts, or solvates thereof, in the manufacture of an antiscarring medicament.

22. The use of a compound according to any one of claims 1 to 18 including the salts or solvates thereof, for the manufacture of a medicament for the treatment of a condition or disease mediated by PCP.

23. A pharmaceutical composition comprising a compound of formula (I), salts thereof, and/or solvates thereof, according to anyone of claims 1 to 18 and a pharmaceutically acceptable diluent, carrier or adjuvant.

24. A process for making a compound of formula (I) wherein W = CZ and the other substituents are as defined in claim 1 and the description related to the processes, which comprises one or more of steps (a) to (d):
(a) reaction of a compound of formula (V), deprotonated if necessary with a base, with a reagent of formula X²SO₂Y¹, where X² is a suitable leaving group, to make a compound of formula (IV); 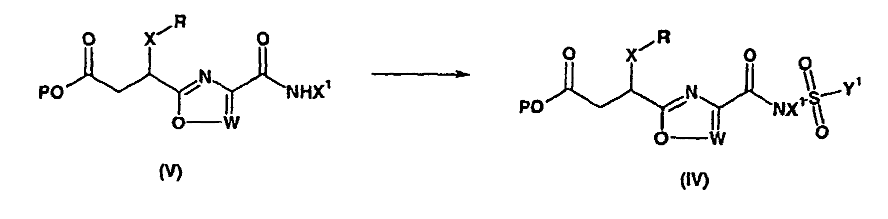
(b) deprotection of a compound of formula (IV) to make a compound of formula (III); 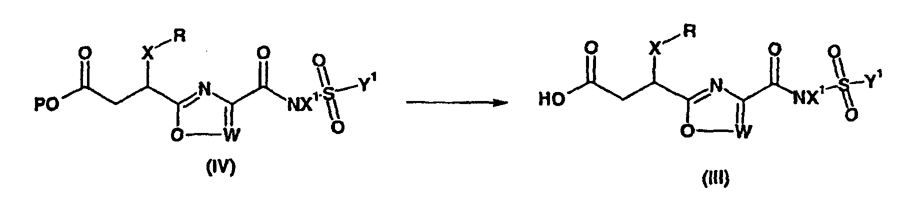
(c) reaction of a compound of formula (III) with coupling agent to form a compound of formula (II) where L¹ is a suitable leaving group; 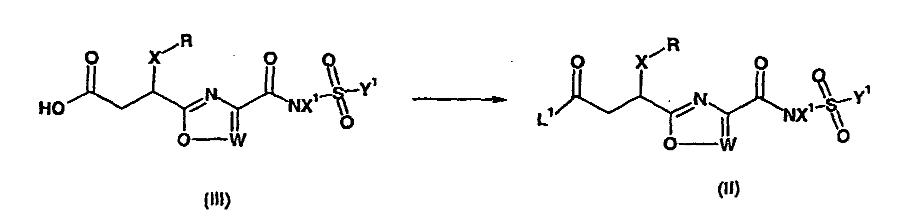 and/or
(d) reaction of a compound of formula (II) with hydroxylamine or a protected derivative thereof to form the hydroxamic acid of formula (I).

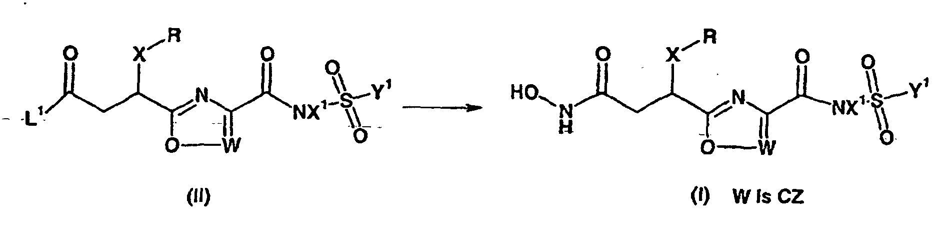

25. A process for making a compound of formula (I) where W = N, and the other substituents are as as defined in claim 1 and the description related to the processes, which comprises one or more of the steps (a) to (d):
(a) reaction of a compound of formula (XII), deprotonated if necessary with a base, with a reagent of formula X²SO₂Y¹, where X² is a suitable leaving group, to make a compound of formula (XI); 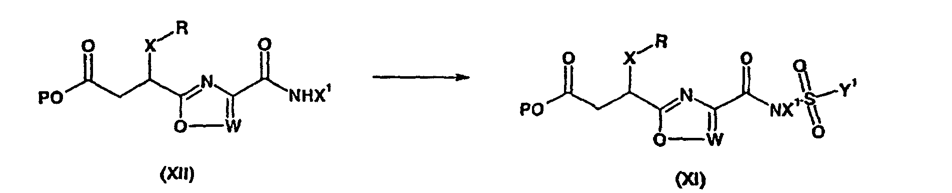
(b) deprotection of a compound of formula (XI) to make a compound of formula (X); 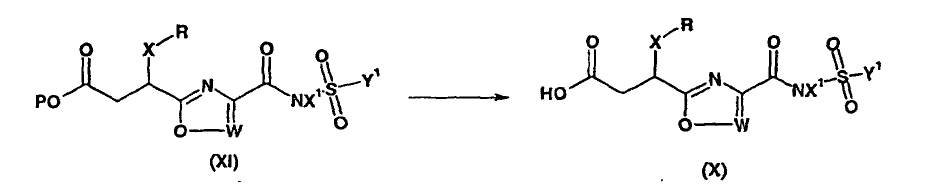
(c) reaction of a compound of formula (X) with a coupling agent, to form a compound of formula (IX) where L¹ is a suitable leaving group; 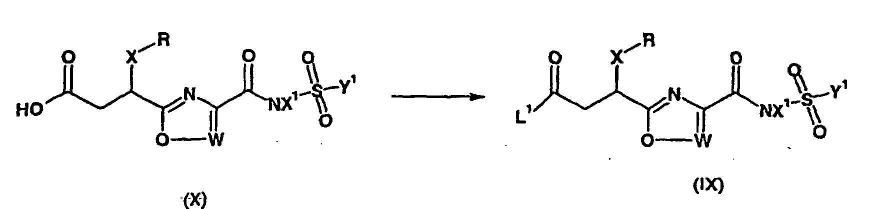 AND/or
(d) reaction of a compound of formula (IX) with hydroxylamine or a protected derivative thereof to form a hydroxamic acid of formula (I).

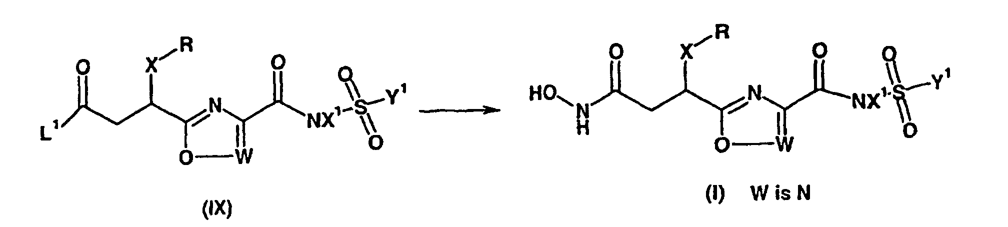

## Patentansprüche

1. Verbindung der Formel I 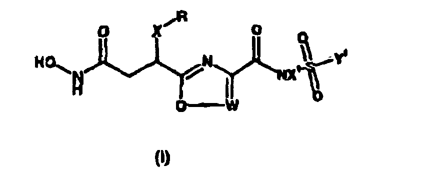 worin
X C₁₋₆-Alkylen oder C₂₋₆-Alkenylen bedeutet, die jeweils optional mit einem oder mehreren Fluoratomen substituiert sind;
R Aryl, C₃₋₈-Cycloalkyl oder C₅₋₈-Cycloalkenyl bedeutet, die optional mit einem oder mehreren Fluoratomen substituiert sind;
W N oder CZ bedeutet;
Z H oder C₁-C₄-Alkyl, das optional mit Halogen substituiert ist, bedeutet;
X¹ H oder C₁-C₄-Alkyl bedeutet;
Y¹ folgendes bedeutet:
C₁-C₄-Alkyl, das optional mit Aryl oder mit einem oder mehreren Halogenatomen substituiert ist, mit der Maßgabe, dass dann, wenn Y¹ Methyl bedeutet, x¹ nicht die Bedeutung H hat,
oder Y¹ Aryl oder einen mono- oder bicyclischen, nicht-aromatischen carbocyclischen oder heterocyclischen Rest bedeutet, der bis zu 10 Ringatome enthält und der bis zu drei Ringheteroatome, die unabhängig voneinander aus N, O und S ausgewählt sind, aufweisen kann, wobei der Ringrest optional mit einem oder mehreren Substituenten substituiert ist, die unabhängig voneinander aus Halogen, C₁-C₄-Alkoxy und C₁-C₄-Alkyl, optional substituiert mit einem oder mehreren Halogenatomen, ausgewählt sind;
"Aryl" einen mono- oder bicyclischen, aromatischen carbocyclischen oder heterocyclischen Rest bedeutet, der bis zu 10 Ringatome enthält und der bis zu drei Ringheteroatome, die unabhängig voneinander aus N, O und S ausgewählt sind, aufweisen kann, wobei der Ringrest optional mit einem oder mehreren Substituenten substituiert ist, die unabhängig voneinander aus Halogen, C₁-C₄-Alkoxy und C₁-C₄-Alkyl, optional substituiert mit Halogen, ausgewählt sind;
und pharmazeutisch verträgliche Salze oder Solvate (einschließlich Hydrate) davon.

2. Verbindung nach Anspruch 1, wobei die Stereochemie der Definition in der Formel (1A) entspricht:

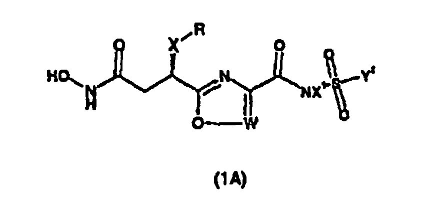

3. Verbindung, Salz oder Solvat nach Anspruch 1 oder 2, wobei X einen linearen C₂₋₄-Alkylenrest bedeutet, der optional mit einem oder mehreren Fluoratomen substituiert ist.

4. Verbindung, Salz oder Solvat nach Anspruch 3, wobei X Propylen bedeutet.

5. Verbindung, Salz oder Solvat nach einem der vorstehenden Ansprüche, wobei R C₃₋₈-Cycloalkyl bedeutet, das optional mit einem oder mehreren Fluoratomen substituiert ist.

6. Verbindung, Salz oder Solvat nach Anspruch 5, wobei R Cyclobutyl oder Cyclohexyl bedeutet, die optional mit einem oder mehreren Fluoratomen substituiert sind.

7. Verbindung, Salz oder Solvat nach Anspruch 6, wobei R Cyclobutyl oder Cyclohexyl bedeutet.

8. Verbindung, Salz oder Solvat nach Anspruch 7, wobei R Cyclohexyl bedeutet.

9. Verbindung, Salz oder Solvat nach einem der vorstehenden Ansprüche, wobei W N, CH oder CCH₃ bedeutet.

10. Verbindung, Salz oder Solvat nach Anspruch 9, wobei W N bedeutet.

11. Verbindung, Salz oder Solvat nach einem der vorstehenden Ansprüche, wobei Y¹ C₁-C₄-Alkyl (optional substituiert mit Phenyl oder mit einem oder mehreren Halogenatomen), Phenyl (optional substituiert mit einem oder mehreren Substituenten, die unabhängig voneinander aus Halogen, C₁-C₄-Alkoxy, C₁-C₄-Alkyl, optional substituiert mit einem oder mehreren Halogenatomen, ausgewählt sind, wobei der Phenylring optional mit einem Pyridinring kondensiert ist), einen 5- oder 6-gliedrigen heterocyclischen Ring (mit einem oder zwei Ringheteroatomen, die aus N, O und S ausgewählt sind, wobei der heterocyclische Ring optional mit einem oder mehreren Substituenten substituiert ist, die unabhängig voneinander aus Halogen, C₁-C₄-Alkoxy, C₁-C₄-Alkyl, optional substituiert mit einem oder mehreren Halogenatomen, ausgewählt sind) bedeutet.

12. Verbindung, Salz oder Solvat nach Anspruch 11, wobei Y¹ Phenyl, 4-Methylphenyl, 4-Methoxyphenyl, 4-Fluorphenyl, 4-Isopropylphenyl, 3,4-Dimethoxyphenyl, 8-Chinolinyl, 3,5-Dimethyl-4-isoxazolyl, Isopropyl, Methyl, Benzyl oder 3-Pyridyl bedeutet.

13. Verbindung, Salz oder Solvat nach Anspruch 12, wobei Y¹ Phenyl, Benzyl, 3,4-Dimethoxyphenyl oder 3-Pyridyl bedeutet.

14. Verbindung, Salz oder Solvat nach Anspruch 13, wobei Y¹ Phenyl bedeutet.

15. Verbindung, Salz oder Solvat nach einem der vorstehenden Ansprüche, wobei X¹ H oder Methyl bedeutet.

16. Verbindung, Salz oder Solvat nach Anspruch 15, wobei X¹ H bedeutet.

17. Verbindung, Salz oder Solvat der Formel (I) nach Anspruch 1, wobei die Substituenten derart sind, wie in den Verbindungen in den hier beschriebenen Beispielen angegeben, und den Salzen oder Solvaten davon.

18. Verbindung nach Anspruch 1, ausgewählt aus:
(3R)-6-Cyclohexyl-N-hydroxy-3-(3-{[(phenylsulfonyl)-amino]-carbonyl}-1,2,4-oxadiazol-5-yl)-hexanamid;
(3R)-6-Cyclohexyl-N-hydroxy-3-[3-({[(4-methylphenyl)-sulfonyl]-amino}-carbonyl)-1,2,4-oxadiazol-5-yl]-hexanamid ;
(3R)-6-Cyclohexyl-N-hydroxy-3-[3-({[(4-methoxyphenyl)-sulfonyl]-amino}-carbonyl)-1,2,4-oxadiazol-5-yl]-hexanamid;
(3R)-6-Cyclohexyl-3-[3-({[(4-fluorphenyl)-sulfonyl]-amino}-carbonyl)-1,2,4-oxadiazol-5-yl]-N-hydroxyhexanamid;
(3R)-6-Cyclohexyl-N-hydroxy-3-[3-({[(4-isopropylphenyl)-sulfonyl]-amino}-carbonyl)-1,2,4-oxadiazol-5-yl]-hexanamid;
(3R)-6-Cyclohexyl-3-[3-({[(3,4-dimethoxyphenyl)-sulfonyl]-amino}-carbonyl)-1,2,4-oxadiazol-5-yl]-N-hydroxyhexanamid;
(3R)-6-Cyclohexyl-N-hydroxy-3-(3-{[(8-chinolinylsulfonyl)-amino]-carbonyl}-1,2,4-oxadiazol-5-yl)-hexanamid;
(3R)-6-Cyclohexyl-3-[3-({[(3,5-dimethyl-4-isoxazolyl)-sulfonyl]-amino}-carbonyl)-1,2,4-oxadiazol-5-yl]-N-hydroxyhexanamid;
(3R)-6-Cyclohexyl-N-hydroxy-3-(3-{[(isopropylsulfonyl)-amino]-carbonyl}-1,2,4-oxadiazol-5-yl)-hexanamid;
(3R)-6-Cyclohexyl-N-hydroxy-3-(3-{[methyl-(methylsulfonyl)-amino]-carbonyl}-1,2,4-oxadiazol-5-yl)-hexanamid;
(3R)-6-Cyclohexyl-N-hydroxy-3-(3-{[(phenylmethyl)-sulfonyl]-amino}-carbonyl)-1,2,4-oxadiazol-5-yl)-hexanamid;
(3R)-6-Cyclohexyl-N-hydroxy-3-(3-{[(3-pyridylsulfonyl)-amino]-carbonyl}-1,2,4-oxadiazol-5-yl)-hexanamid; und die Salze und Solvate davon.

19. Verbindung nach einem der Ansprüche 1 bis 18, einschließlich der Salze oder Solvate davon, zur Verwendung in der Medizin.

20. Verbindung nach einem der Ansprüche 1 bis 18, einschließlich der Salze oder Solvate davon, zur Verwendung bei der Behandlung eines PCP-vermittelten Zustands oder Krankheit.

21. Verwendung einer Verbindung nach einem der Ansprüche 1 bis 18, einschließlich der Salze oder Solvate davon, bei der Herstellung eines Arzneimittels gegen Narbenbildung.

22. Verwendung einer Verbindung nach einem der Ansprüche 1 bis 18, einschließlich der Salze oder Solvate davon, bei der Herstellung eines Arzneimittels zur Behandlung eines Zustands oder einer Krankheit, die durch PCP vermittelt sind.

23. Pharmazeutische Zusammensetzung, umfassend eine Verbindung der Formel (I), Salze und/oder Solvate davon, nach einem der Ansprüche 1 bis 18 und ein pharmazeutisch akzeptables Verdünnungsmittel, Trägerstoff oder Adjuvans.

24. Verfahren zur Herstellung einer Verbindung der Formel (I), wobei W=CZ und die übrigen Substituenten den in Anspruch 1 und in der Verfahrensbeschreibung der Verfahren angegebenen Definitionen entsprechen, wobei das Verfahren eine oder mehrere der Stufen (a) bis (d) umfasst.
(a) Umsetzung einer Verbindung der Formel (V), die gegebenenfalls mit einer Base deprotoniert ist, mit einem Reagenz der Formel X²SO₂Y¹, wobei X² eine geeignete austretende Gruppe bedeutet, unter Bildung einer Verbindung der Formel (IV); 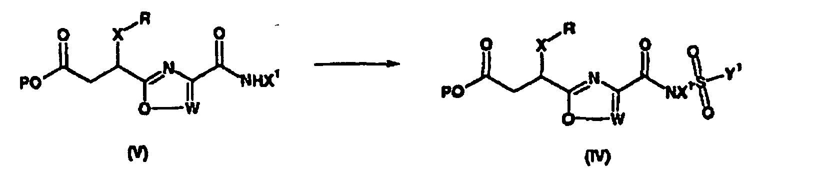
(b) Schutzgruppenentfernung von einer Verbindung der Formel (IV) unter Bildung einer Verbindung der Formel (III); 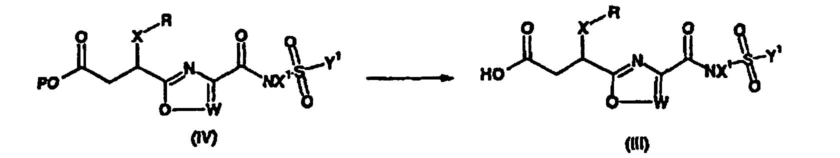
(c) Umsetzung einer Verbindung der Formel (III) mit einem Kupplungsmittel unter Bildung einer Verbindung der Formel (II), wobei L¹ eine geeignete austretende Gruppe bedeutet; 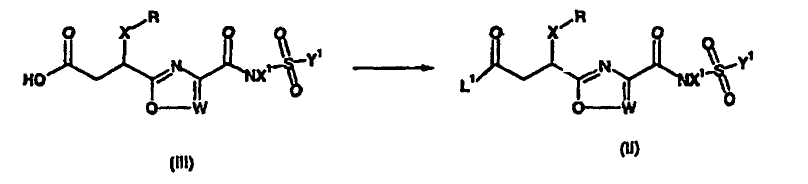 und/oder
(d) Umsetzung einer Verbindung der Formel (II) mit Hydroxylamin oder einem geschützten Derivat davon unter Bildung einer Hydroxamsäure der Formel (I)

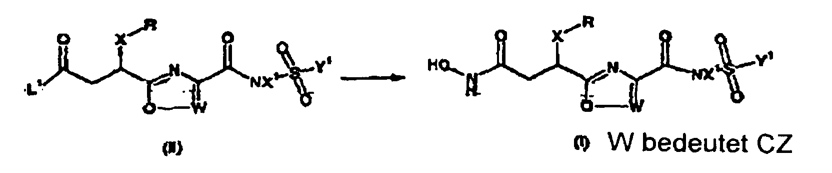

25. Verfahren zur Herstellung einer Verbindung der Formel (I), wobei W=N und die übrigen Substituenten den in Anspruch 1 und in der Verfahrensbeschreibung der Verfahren angegebenen Definitionen entsprechen, wobei das Verfahren eine oder mehrere der Stufen (a) bis (d) umfasst:
(a) Umsetzung einer Verbindung der Formel (XII), die gegebenenfalls mit einer Base deprotoniert ist, mit einem Reagenz der Formel X²SO₂Y¹, wobei X² eine geeignete austretende Gruppe bedeutet, unter Bildung einer Verbindung der Formel (XI); 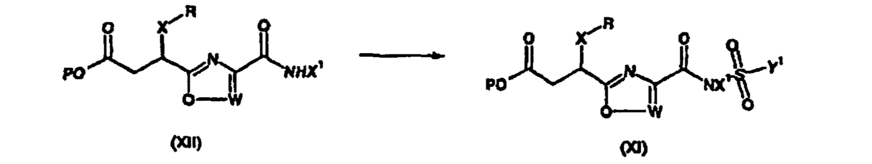
(b) Schutzgruppenentfernung von einer Verbindung der Formel (XI) unter Bildung einer Verbindung der Formel (X); 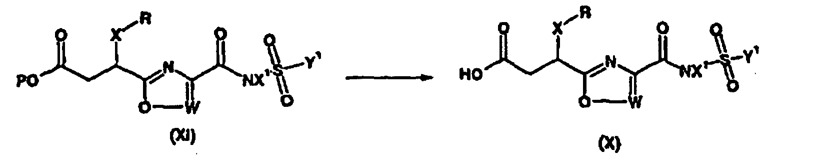
(c) Umsetzung einer Verbindung der Formel (X) mit einem Kupplungsmittel unter Bildung einer Verbindung der Formel (IX), wobei L¹ eine geeignete austretende Gruppe bedeutet; 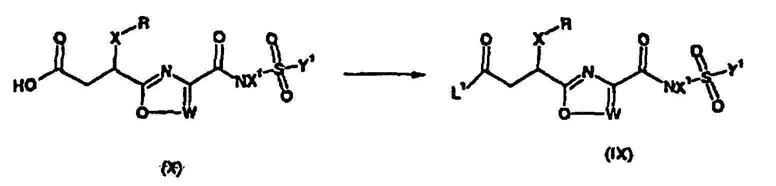 und/oder
(d) Umsetzung einer Verbindung der Formel (IX) mit Hydroxylamin oder einem geschützten Derivat davon unter Bildung einer Hydroxamsäure der Formel (I)

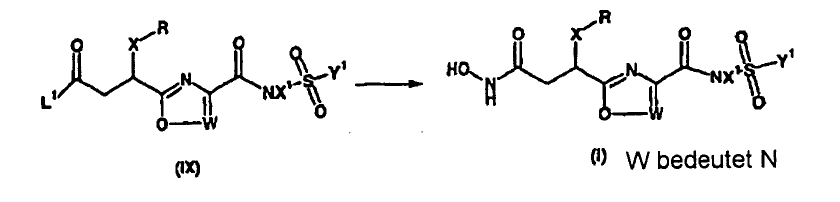

## Revendications

1. Composé de formule I : 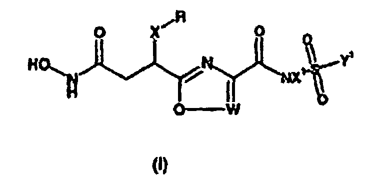 dans laquelle :
X représente un groupe alkylène en C₁ à C₆ ou alcénylène en C₂ à C₆, dont chacun est facultativement substitué avec un ou plusieurs atomes de fluor ;
R représente un groupe aryle, cycloalkyle en C₃ à C₈ ou cycloalcényle en C₅ à C₈ facultativement substitué avec un ou plusieurs atomes de fluor ;
W représente un atome de N ou un groupe CZ ;
Z représente H, ou un groupe alkyle en C₁ à C₄ facultativement substitué avec un halogène ;
X¹ représente H ou un groupe alkyle en C₁ à C₄ ;
Y¹ représente :
un groupe alkyle en C₁ à C₄ facultativement substitué avec un substituant aryle, ou avec un ou plusieurs atomes d'halogènes, sous réserve que, lorsque Y¹ représente un groupe méthyle, X¹ ne représente pas H ;
ou bien Y¹ représente un groupe aryle, ou un groupement carbocyclique ou hétérocyclique non aromatique mono- ou bicyclique contenant jusqu'à 10 atomes dans les noyaux et qui peut comprendre dans les noyaux jusqu'à 3 hétéroatomes, choisis indépendamment entre N, O et S, groupement cyclique qui est facultativement substitué avec un ou plusieurs substituants choisis indépendamment entre des substituants halogéno, alkoxy en C₁ à C₄ et alkyle en C₁ à C₄ facultativement substitué avec un ou plusieurs substituants halogéno ;
le groupe "aryle" est un groupement carbocyclique ou hétérocyclique aromatique mono- ou bicyclique contenant jusqu'à 10 atomes dans les noyaux, et qui peut comprendre dans les noyaux jusqu'à 3 hétéroatomes, choisis indépendamment entre N, O et S, groupement cyclique qui est facultativement substitué avec un ou plusieurs substituants choisis indépendamment entre des substituants halogéno, alkoxy en C₁ à C₄ et alkyle en C₁ à C₄ facultativement substitué avec des substituants halogéno ;
et ses sels ou produits de solvatation (y compris hydrates) pharmaceutiquement acceptables.

2. Composé suivant la revendication 1, dans lequel la stéréochimie est telle que définie dans la formule (IA) :

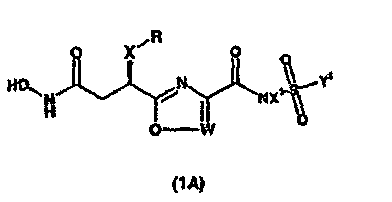

3. Composé, sel ou produit de solvatation suivant la revendication 1 ou la revendication 2, dans lequel X représente un groupement alkylène en C₂ à C₄ linéaire facultativement substitué avec un ou plusieurs atomes de fluor.

4. Composé, sel ou produit de solvatation suivant la revendication 3, dans lequel X représente un groupe propylène.

5. Composé, sel ou produit de solvatation suivant l'une quelconque des revendications précédentes, dans lequel R représente un groupe cycloalkyle en C₃ à C₈ facultativement substitué avec un ou plusieurs atomes de fluor.

6. Composé, sel ou produit de solvatation suivant la revendication 5, dans lequel R représente un groupe cyclobutyle ou cyclohexyle facultativement substitué avec un ou plusieurs atomes de fluor.

7. Composé, sel ou produit de solvatation suivant la revendication 6, dans lequel R représente un groupe cyclobutyle ou cyclohexyle.

8. Composé, sel ou produit de solvatation suivant la revendication 7, dans lequel R représente un groupe cyclohexyle.

9. Composé, sel ou produit de solvatation suivant l'une quelconque des revendications précédentes, dans lequel W représente un atome de N, un groupe CH ou CCH₃.

10. Composé, sel ou produit de solvatation suivant la revendication 9, dans lequel W représente un atome de N.

11. Composé, sel ou produit de solvatation suivant l'une quelconque des revendications précédentes, dans lequel Y¹ représente un groupe alkyle en C₁ à C₄ (facultativement substitué avec un substituant phényle ou avec un ou plusieurs atomes d'halogènes), phényle (facultativement substitué avec un ou plusieurs substituants choisis indépendamment entre des substituants halogéno, alkoxy en C₁ à C₄, alkyle en C₁ à C₄ facultativement substitué avec un ou plusieurs substituants halogéno, ledit noyau phényle étant facultativement pyrido-condensé), un noyau hétérocyclique penta- ou hexagonal (avec un ou deux hétéroatomes de noyau choisis entre N, O et S, noyau hétérocyclique qui est facultativement substitué avec un ou plusieurs substituants choisis indépendamment entre des substituants halogéno, alkoxy en C₁ à C₄, alkyle en C₁ à C₄ facultativement substitué avec un
ou plusieurs atomes d'halogènes).

12. Composé, sel ou produit de solvatation suivant la revendication 11, dans lequel Y¹ représente un groupe phényle, méthylphényle, 4-méthoxyphényle, 4-fluorophényle, 4-isopropylphényle, 3,4-diméthoxyphényle, 8-quinolinyle, 3,5-diméthyl-4-isoxazolyle, isopropyle, méthyle, benzyle ou 3-pyridyle.

13. Composé, sel ou produit de solvatation suivant la revendication 12, dans lequel Y¹ représente un groupe phényle, benzyle, 3,4-diméthoxyphényle ou 3-pyridyle.

14. Composé, sel ou produit de solvatation suivant la revendication 13, dans lequel Y¹ représente un groupe phényle.

15. Composé, sel ou produit de solvatation suivant l'une quelconque des revendications précédentes, dans lequel X¹ représente H ou un groupe méthyle.

16. Composé, sel ou produit de solvatation suivant la revendication 15, dans lequel X¹ représente H.

17. Composé, sel ou produit de solvatation de formule (I) suivant la revendication 1, dans lequel les substituants sont tels que spécifiés dans les composés des exemples décrits dans le présent mémoire et leurs sels ou produits de solvatation.

18. Composé suivant la revendication 1, qui est choisi entre :
(3R)-6-cyclohexyl-N-hydroxy-3-(3-{[(phénylsulfonyl)-amino]carbonyl}-1,2,4-oxadiazole-5-yl)-hexanamide ;
(3R)-6-cyclohexyl-N-hydroxy-3-[3-({[(4-méthylphényl)-sulfonyl]amino}carbonyl)-1,2,4-oxadiazole-5-yl]-hexanamide ;
(3R)-6-cyclohexyl-N-hydroxy-3-[3-({[(4-méthoxyphényl)-sulfonyl]amino}carbonyl)-1,2,4-oxadiazole-5-yl]-hexanamide ;
(3R)-6-cyclohexyl-3-[3-({[(4-fluorophényl)sulfonyl]-amino}carbonyl)-1,2,4-oxadiazole-5-yl]-N-hydroxy-hexanamide ;
(3R)-6-cyclohexyl-N-hydroxy-3-[3-({[(4-isopropyl-phényl)sulfonyl]amino}carbonyl)-1,2,4-oxadiazole-5-yl]-hexanamide ;
(3R)-6-cyclohexyl-3-[3-({[(3,4-diméthoxyphényl)-sulfonyl]amino}carbonyl)-1,2,4-oxadiazole-5-y]-N-hydroxy-hexanamide ;
(3R)-6-cyclohexyl-N-hydroxy-3-(3-{[(8-quinolinylsulfonyl)amino]carbonyl}-1,2,4-oxadiazole-5-yl)-hexanamide ;
(3R)-6-cyclohexyl-3-[3-({[(3,5-diméthyl-4-isoxazolyl)-sulfonyl]amino}carbonyl)-1,2,4-oxadiazole-5-yl]-N-hydroxy-hexanamide ;
(3R)-6-cyclohexyl-N-hydroxy-3-(3-{[(isopropylsulfonyl)amino]carbonyl}-1,2,4-oxadiazole-5-yl)-hexanamide ;
(3R)-6-cyclohexyl-N-hydroxy-3-(3-{[méthyl(méthylsulfonyl)amino]carbonyl}-1,2,4-oxadiazole-5-yl]-hexanamide ;
(3R)-6-cyclohexyl-N-hydroxy-3-(3-{[(phénylméthyl)-sulfonyl]amino}carbonyl)-1,2,4-oxadiazole-5-yl)-hexanamide ;
(3R)-6-cyclohexyl-N-hydroxy-3-(3-{[(3-pyridylsulfonyl)amino]carbonyl}-1,2,4-oxadiazole-5-yl)-hexanamide ;
et leurs sels ou produits de solvatation.

19. Composé suivant l'une quelconque des revendications 1 à 18, y compris ses sels ou produits de solvatation, destinés à être utilisés en médecine.

20. Composé suivant l'une quelconque des revendications 1 à 18, y compris ses sels ou produits de solvatation, destinés à être utilisés dans le traitement d'une affection ou maladie à médiation par PCP.

21. Utilisation d'un composé suivant l'une quelconque des revendications 1 à 18, y compris ses sels ou produits de solvatation, dans la production d'un médicament anti-cicatrisant.

22. Utilisation d'un composé suivant l'une quelconque des revendications 1 à 18, y compris ses sels ou produits de solvatation, pour la production d'un médicament destiné au traitement d'une affection ou maladie à médiation par PCP.

23. Composition pharmaceutique comprenant un composé de formule (I), y compris ses sels et/ou produits de solvatation, suivant l'une quelconque des revendications 1 à 18, et un diluant, support ou adjuvant pharmaceutiquement acceptable.

24. Procédé pour la préparation d'un composé de formule (I) dans laquelle W représente un groupe CZ et les autres substituants sont tels que définis dans la revendication 1 et la description en rapport avec les procédés, qui comprend une ou plusieurs des étapes (a) à (d) :
(a) réaction d'un composé de formule (V), déprotoné si nécessaire avec une base, avec un réactif de formule X²SO₂Y¹, dans laquelle X² représente un groupe partant convenable, pour préparer un composé de formule (IV) : 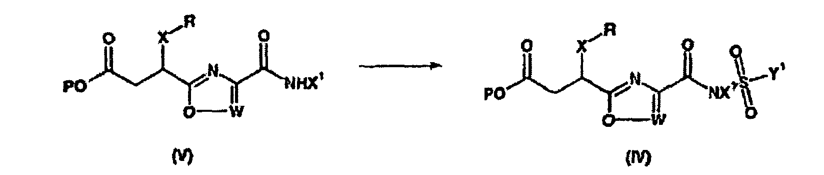
(b) suppression de la protection d'un composé de formule (IV) pour préparer un composé de formule (III) : 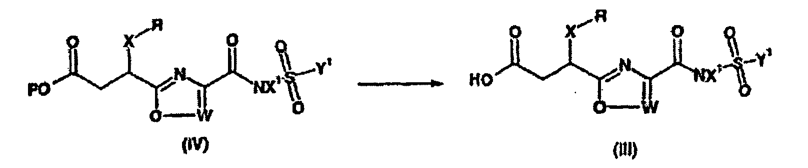
(c) réaction d'un composé de formule (III) avec un agent de couplage pour former un composé de formule (II) dans lequel L¹ représente un groupe partant convenable : 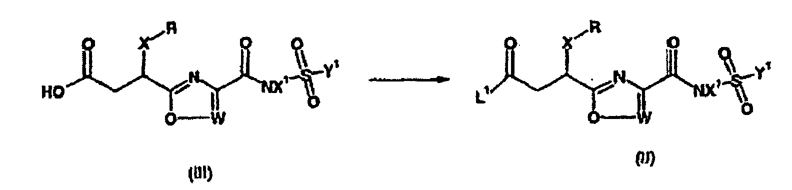 et/ou
(d) réaction d'un composé de formule (II) avec de l'hydroxylamine ou un de ses dérivés protégés pour former l'acide hydroxamique de formule (I)

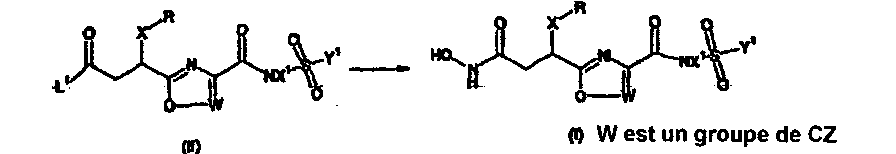

25. Procédé pour la préparation d'un composé de formule (I) dans laquelle W représente un atome de N et les autres substituants sont tels que définis dans la revendication 1 et la description en rapport avec les procédés, qui comprend une ou plusieurs des étapes (a) à (d) :
(a) réaction d'un composé de formule (XII), déprotoné si nécessaire avec une base, avec un réactif de formule X²SO₂Y¹, dans laquelle X² représente un groupe partant convenable, pour préparer un composé de formule (XI) : 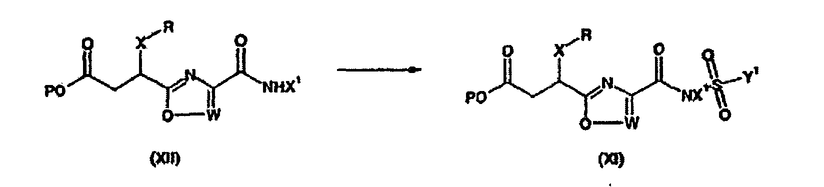
(b) suppression de la protection d'un composé de formule (XI) pour préparer un composé de formule (X) : 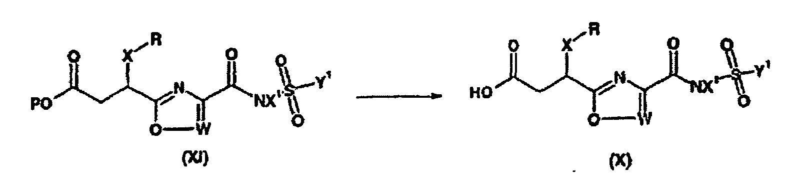
(c) réaction d'un composé de formule (X) avec un agent de couplage pour former un composé de formule (IX) dans laquelle L¹ représente un groupe partant convenable :
(c) réaction d'un composé de formule (X) avec un agent de couplage pour former un composé de formule (IX) dans laquelle L¹ représente un groupe partant convenable : 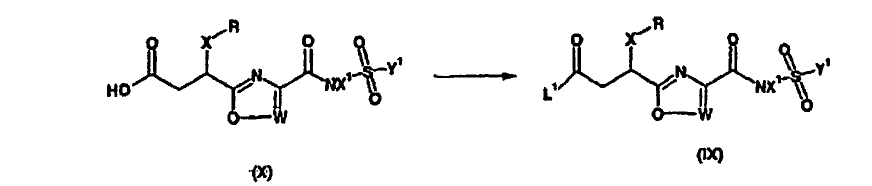 et/ou
(d) réaction d'un composé de formule (IX) avec l'hydroxylamine ou un de ses dérivés protégés pour former un acide hydroxamique de formule (I)
